# EUROPEAN PATENT APPLICATION

(11) **EP 1 186 588 A1**
(43) Date of publication of application: **13.03.2002**
(21) Application number: 00929861.3
(22) Date of filing: 25.05.2000
(51) Int. Cl.: C07C 39/14, C07C 29/147, C07C 47/57, C07C 45/29

(54) **6-HYDROXY-2-NAPHTHYLCARBINOL AND PROCESS FOR THE PREPARATION THEREOF**

(30) Priority: 26.05.1999 JP 14643799; 26.05.1999 JP 14643899; 29.09.1999 JP 27609199
(71) Applicant: MITSUBISHI CHEMICAL CORPORATION, Chiyoda-ku, Tokyo 100-0005 (JP)
(72) Inventor: QU, Jingping, Mitsubishi Chemical Corporation, Inashiki-gun, Ibaraki 300-03 (JP); ICHIKAWA, Shuji, Mitsubishi Chemical Corporation, Inashiki-gun, Ibaraki 300-0332 (JP); IWANE, Hiroshi, Mitsubishi Chemical Corporation, Inashiki-gun, Ibaraki 300-0332 (JP)
(74) Representative: Sternagel, Fleischer, Godemeyer & Partner Patentanwälte
(86) International application number: JP0003343
(87) International publication number: WO0073252

(57) **Abstract**

6-Hydroxy-2-naphthylcarbinol useful as a synthetic intermediate for preparation of 6-hydroxy-2-naphthaldehyde represented by the following formula (I), and a method for preparing 6-hydroxy-2-naphthylcarbinol comprising the step of reducing 6-hydroxy-2-naphthalenecarboxylic acid.

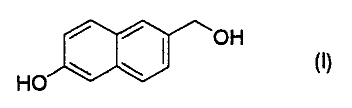

## Description

### Technical Field

The present invention relates to a hydroxynaphthylcarbinol, in particular, 6-hydroxy-2-naphthylcarbinol (this compound is also referred to as 6-hydroxy-2-naphthalenemethanol"), which is useful as a synthetic intermediate for preparation of pharmaceuticals, agricultural chemicals and the like.

### Background Art

Japanese Patent No. 2845743 and Japanese Patent Unexamined Publication (Kokai) No. 10-139768 disclose 5-(6-substituted benzyloxy-2-naphthyl]-methyl-2,4-thiazolidinediones represented by the following general formula (X): 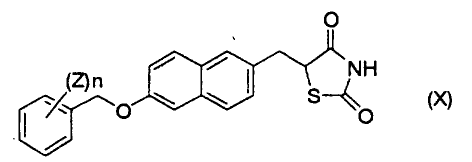 wherein Z represents a halogen atom and n represents an integer of 1-5. These compounds are known to have hypoglycemic as well as hypolipidemic action of blood lipids, and knows to be useful as medicaments for prophylactic and/or therapeutic treatment of diabetes mellitus and hyperlipidemia.

The aforementioned publications discloses that 6-hydroxy-2-naphthaldehyde represented by the following formula was reacted with 2,4-thiazolidinedione, and the resulting product was reduced and then reacted with a compound represented by the general following formula (IX) to produce the target compounds. However, the publications fail to specifically disclose the method for preparation of 6-hydroxy-2-naphthaldehyde.

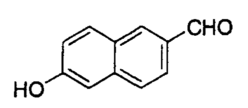

6-Hydroxy-2-naphthaldehyde is usually produced by the methods mentioned below. For example, reported methods include a method of dealkylating 6-alkoxy-2-naphthaldehyde in the presence of a Lewis acid such as aluminum chloride and potassium iodide as an auxiliary agent (Japanese Patent Unexamined Publication No. 9-255613) or in a chlorinated solvent (Japanese Patent Unexamined Publication No. 9-255612), a method of dealkylating said compound by using aluminum iodide as a Lewis acid (Japanese Patent Unexamined Publication No. 9-59202), a method of reducing 6-hydroxy-2-naphthonitrile by using diisobutyl aluminum hydride (Japanese Patent Unexamined Publication No. 10-147568), a method comprising the steps of brominating 3,4-dimethylphenyl benzoic acid ester by using N-bromosuccinimide, then converting the resulting product into a diene compound by dehydrobromination using sodium iodide, and then subjecting the resulting product to Diels-Alder reaction using acrolein to produce the target compound (J. Org. Chem., USSR., 17, 303 (1981)) and the like. However, in these methods, multiple steps and expensive regents are required for preparation of the raw materials to be used, and no satisfactory yield can be achieved since the target compound is a substrate having highly reactive formyl group. Therefore, they are not suitable as industrial applicable preparations.

There have also been proposed methods comprising oxidizing an alcohol in the presence of a stable nitroxy radical and nitrosodisulfonic acid alkali metal salt (U.S. Patent No. 5,155,280) or nitrous acid, nitric acid or an-alkali metal salt thereof (U.S. Patent No. 5,155,278) and in the presence of oxygen and water to produce a corresponding aldehyde. However, when a reaction for conversion of hydroxynaphthalenemethanol to hydroxynaphthaldehyde is carried out according to these methods, target compounds are obtained in a low yield.

Methods for producing naphthylmethylthiazolidinedione derivatives are disclosed in Japanese Patent Unexamined Publication No. 6-247945 (Japanese Patent No. 2845743). One of the methods comprises the steps of introducing a substituent to 6-acetyl-2-naphthol at its hydroxyl group, converting the resulting product into naphthylamine through the Beckmann rearrangement and further into a diazonium salt, preparing a halo ester by the Meerwein arylation, and then reacting the resulting product with thiourea to produce iminothiazolidinone, and further hydrolyzing the iminothiazolidinone under an acidic condition to produce a naphthylmethylthiazolidinedione derivative. This preparation method contains multiple steps, and has drawbacks such as heavy exothermic heat and generation of a huge volume of nitrogen gas in the Meewein arylation and mephitis due to the use of acrylic ester. Furthermore, if the substituent of the hydroxyl group on the naphthalene ring is benzyl group, the benzyl group may be eliminated depending on the kind of a substituent on the benzyl group in the hydrolysis from the iminothiazolidinone to the thiazolidinedione under an acid condition.

Another preparation method is disclosed which comprises the steps of introducing a substituent into the hydroxyl group of 6-hydroxy-2-naphthaldehyde and then reducing the formyl group into hydroxyl group, and the converting the resulting product to a naphthylmethyl halide by using a halogenating agent and reacting the naphthylmethyl halide with dianion of thiazolidinedione at a low temperature to produce naphthylmethylthiazolidinedione. In the preparation method, the naphthylmethyl halide produced as an intermediate is unstable and the alkylation of thiazolidinedione with the dianion is only conducted at a low temperature. Therefore, this method also has problems for the production in an industrial scale.

Still another production method is disclosed which comprises the steps of protecting the hydroxyl group of 6-hydroxy-2-naphthaldehyde with a protective group and condensing the resulting product with thiazolidinedione by dehydration, and then reducing the produced double bond into a single bond by a catalytic hydrogenation, followed by eliminating the protective group and introducing a substituent site-specifically to the phenolic hydroxyl group. However, this production method contains the steps of protection and deprotection of the phenolic hydroxyl group, and therefore is not a reasonable production method.

### Disclosure of the Invention

An object of the present invention is to provide a synthetic intermediate that enables efficient preparation of 6-hydroxy-2-naphthaldehyde, which is useful as a raw material for the manufacture of pharmaceutical compounds, agrichemical compounds and the like.

Another object of the present invention is to provide a method for producing the aforementioned synthetic intermediate and a method for efficiently preparing 6-hydroxy-2-naphthaldehyde by using the aforementioned method.

The inventors of the present invention conducted various studies to achieve the aforementioned objects. As a result, they found that 6-hydroxy-2-naphthylcarbinol represented by the following formula (I) was useful as a synthetic intermediate for the preparation of 6-hydroxy-2-naphthaldehyde. The present invention was achieved on the basis of the above finding.

The present invention thus provides 6-hydroxy-2-naphthylcarbinol represented by following formula (I): 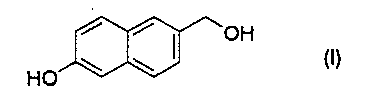 This compound is useful as a synthetic intermediate for the preparation of 6-hydroxy-2-naphthaldehyde. The present invention also provides 6-hydroxy-2-naphthylcarbinol represented by the aforementioned formula (I) used as a synthetic intermediate for the preparation of 6-hydroxy-2-naphthaldehyde.

From another aspect, provided is a method for preparing 6-hydroxy-2-naphthylcarbinol represented by the aforementioned formula (I), which comprises the step of reducing 6-hydroxy-2-naphthalenecarboxylic acid represented by following formula (II):

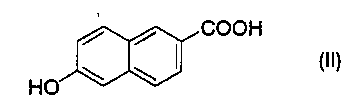

As a preferred embodiment of the aforementioned method, provided is the method in which the reduction is performed by using a borane, and as a more preferred embodiment, the method is provided in which the reduction is performed by using a borane in the presence of a borate represented by the following general formula (III): 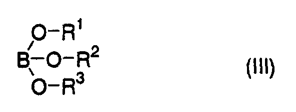 (in the formula, R¹, R² and R³ independently represent an alkyl group, an aralkyl group, a hetero atom-containing alkyl group, or an aryl group); the following general formula (IV): 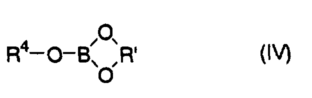 (in the formula, R⁴ represents an alkyl group, an aralkyl group, a hetero atom-containing alkyl group, or an aryl group, and R' represents an alkylene group which may be substituted); the following general formula (V): 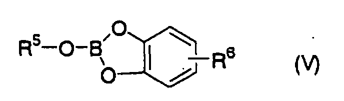 (in the formula, R⁵ and R⁶ independently represent an alkyl group, an aralkyl group, a hetero atom-containing alkyl group, or an aryl group); or the following general formula (VI): 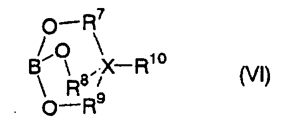 (in the formula, R⁷, R⁸ and R⁹ independently represent an alkylene group which may be substituted or an alkyleneoxy group which may be substituted, R¹⁰ represents a hydrogen atom or an alkyl group, X represents a carbon atom, a nitrogen atom, or a boron atom, and a, b, c and d each independently represent 0 or 1). According to a further preferred embodiment of the aforementioned method, the borate is trimethyl borate or triethyl borate. According to another preferred embodiment of the aforementioned method, the method is provided in which the reduction reaction is performed by using an aluminum hydride complex compound.

From still another aspect of the present invention, provided is a method for producing 6-hydroxy-2-naphthaldehyde, which comprises the step of oxidizing 6-hydroxy-2-naphthylcarbinol represented by the formula (I). According to a preferred embodiment of the aforementioned method, a reducing product of 6-hydroxy-2-naphthalenecarboxylic acid represented by the formula (II) can be used as the 6-hydroxy-2-naphthylcarbinol.

According to a preferred embodiment of the aforementioned method, the method is provided in which the oxidation is performed in the presence of manganese dioxide, preferably activated manganese dioxide. According to another preferred embodiment, provided is the method in which the oxidation is performed by using oxygen in the presence of a catalyst system containing a nitroxide represented by the following general formula (VII): 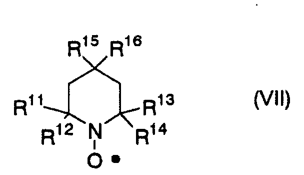 (in the formula, R¹¹, R¹², R¹³ and R¹⁴ independently represent an alkyl group, an aryl group, or a hetero-atom substituted alkyl group, and R¹⁵ and R¹⁶ independently represent a hydrogen atom, an alkyl group, an aryl group, or a hetero atom-containing substituent; or the following general formula (VIII): 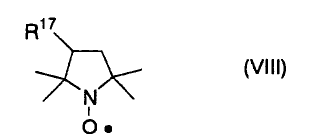 (in the formula, R¹⁷ represents a hydrogen atom, a hydroxyl group, an alkyl group, an alkoxycarbonyl group, a cyano group, a carbamoyl group, an alkoxy group, or an acyloxy group) together with a metal compound having oxidation-reduction ability or a metal complex compound having oxidation-reduction ability. The nitroxide is preferably 2,2,6,6-tetramethylpiperidine-1-oxyl or 4-hydroxy-2,2,6,6-tetramethylpiperidine-1-oxyl, and a metal component of the metal compound having oxidation-reduction ability or the metal complex compound having oxidation-reduction ability is preferably copper or iron.

The present invention further provides a method for producing a compound represented by the following general formula (X): 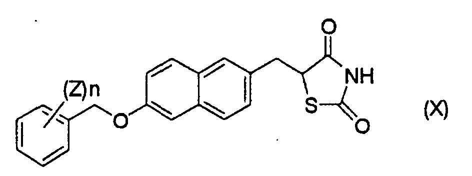 (in the formula, Z represents a halogen atom, and n represents an integer of 1 to 5), which comprises the steps of:
(1) reacting 6-hydroxy-2-naphthaldehyde obtained by oxidizing 6-hydroxy-2-naphthylcarbinol represented by the formula (I) with 2,4-thiazolidinedione in the absence of a protective group for the hydroxyl group of the 6-hydroxy-2-naphthaldehyde to produce 5-(6-hydroxy-2-naphthyl)methylene-2,4-thiazolidinedione;
(2) reducing the 5-(6-hydroxy-2-naphthyl)methylene-2,4-thiazolidinedione obtained in the above step (1) to produce 5-(6-hydroxy-2-naphthyl)methyl-2,4-thiazolidinedione; and
(3) reacting the 5-(6-hydroxy-2-naphthyl)methyl-2,4-thiazolidinedione obtained in the above step (2) with a compound represented by the following general formula (IX): 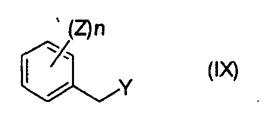 (in the formula, Y represents a halogen atom, an alkylsulfonyloxy group, or an arylsulfonyloxy group, and Z and n have the same meanings as those defined above).

According to a preferred embodiment of the above method, the method is provided wherein 6-hydroxy-2-naphthaldehyde and 2,4-thiazolidinedione are subjected to dehydration condensation in the presence of a base to obtain 5-(6-hydroxy-2-naphthyl)methylene-2,4-thiazolidinedione.

### Best Mode for Carrying out the Invention

6-Hydroxy-2-naphthylcarbinol of the present invention represented by the formula (I) is a novel compound.

The methods of the present invention include:
(A) the method for producing 6-hydroxy-2-naphthylcarbinol represented by the aforementioned formula (I), which comprises the step of reducing 6-hydroxy-2-naphthalenecarboxylic acid represented by the aforementioned formula (II);
(B) the method for producing 6-hydroxy-2-naphthaldehyde, which comprises the step of oxidizing a compound represented by the aforementioned formula (I);
(C) the method for producing 6-hydroxy-2-naphthaldehyde, which comprises the step of oxidizing 6-hydroxy-2-naphthylcarbinol represented by the aforementioned formula (I) which is obtained by reducing the 6-hydroxy-2-naphthalenecarboxylic acid represented by the aforementioned formula (II);
(D) the method for producing a compound represented by the aforementioned general formula (X), which comprises the steps of:
   (1) oxidizing 6-hydroxy-2-naphthylcarbinol represented by the aforementioned formula (I) to produce 6-hydroxy-2-naphthaldehyde;
   (2) reacting the 6-hydroxy-2-naphthaldehyde obtained in the aforementioned step (1) with 2,4-thiazolidinedione;
   (3) reducing the compound obtained in the aforementioned step (2); and
   (4) reacting the compound obtained in the aforementioned step (3) with a compound represented by the aforementioned general formula (IX); and
(E) the method for producing a compound represented by the aforementioned general formula (X), which comprises the steps of:
   (5) reducing 6-hydroxy-2-naphthalenecarboxylic acid represented by the aforementioned formula (II) to produce 6-hydroxy-2-naphthylcarbinol represented by the aforementioned formula (I);
   (6) oxidizing the 6-hydroxy-2-naphthylcarbinol represented by the aforementioned formula (I) obtained in the aforementioned step (5) to produce 6-hydroxy-2-naphthaldehyde;
   (7) reacting the 6-hydroxy-2-naphthaldehyde obtained in the aforementioned step (6) with 2,4-thiazolidinedione;
   (8) reducing the compound obtained in the aforementioned step (7); and
   (9) reacting the compound obtained in the aforementioned step (8) with a compound represented by the aforementioned general formula (IX).

The method for producing 6-hydroxy-2-naphthylcarbinol represented by the formula (I) from 6-hydroxy-2-naphthalenecarboxylic acid represented by the formula (II), the method for producing 6-hydroxy-2-naphthaldehyde from 6-hydroxy-2-naphthylcarbinol represented by the formula (I), and the method for producing a compound represented by the general formula (X) from 6-hydroxy-2-naphthaldehyde will be explained below. However, the methods of the present invention are not limited to the specific preparation methods mentioned below. Further, it should be understood that reaction conditions and reagents can be suitably chosen by those skilled in the art, and suitable alterations and modifications can be made to the methods described below.

The method for producing the novel compound represented by the aforementioned formula (I) of the present invention, 6-hydroxy-2-naphthylcarbinol; will be explained.

6-Hydroxy-2-naphthalenecarboxylic acid of the formula (II) used as the raw material is obtained by, for example, carboxylating potassium salt of β-naphthol through a reaction with carbon dioxide at a high temperature under positive pressure (Kolbe-Schmitt's reaction) and isomerizing the carboxylated product. This compound is also readily obtainable since the compound is commercially produced as a synthetic raw material for liquid crystal polymers. By reducing this 6-hydroxy-2-naphthalenecarboxylic acid, 6-hydroxy-2-naphthylcarbinol of the formula (I) can be obtained.

The reducing agent is not particularly limited so long as the agent can reduce carboxyl group into an alcohol and is inert to naphthalene ring. For example, examples include boranes, aluminum hydride complex compounds and the like.

Specific examples of the borane include diborane, borane/tetrahydrofuran complex, borane/dimethyl sulfide complex, borane/1,4-oxathiane complex, borane/dimethylaniline complex, borane/diethylaniline complex, borane/4-phenylmorpholine complex, catecholborane, texylborane, disiamylborane, alkylboranes such as 9-borabicyclo[3.3.1]nonane and the like. Among them, borane/tetrahydrofuran complex, borane/dimethyl sulfide complex and borane/diethylaniline complex are preferred, and borane/tetrahydrofuran complex is particularly preferred. These boranes may be used in combination.

As the boranes, commercially available products, per se, can be used, or they may be generated in a system by using a borohydride compound and then used. For example, the borane/tetrahydrofuran complex can be easily prepared by reacting a borohydride compound with dimethyl sulfate or boron trifluoride in tetrahydrofuran (THF) as a solvent.

As the borohydride compound, sodium borohydride, lithium borohydride, calcium borohydride, zinc borohydride, magnesium borohydride, potassium borohydride, beryllium borohydride, barium borohydride and the like may be used. Sodium borohydride, lithium borohydride, calcium borohydride and the like are preferred mainly from an economical standpoint.

The borane can be used generally about 1 to 8 mole, preferably about 1.3 to 4 mole based on 6-hydroxy-2-naphthalenecarboxylic acid. If the borane is used in an amount less than equimolar amount, the reaction of 6-hydroxy-2-naphthalenecarboxylic acid is incomplete, and an amount over 8 mole is not preferred mainly from an economical standpoint.

Specific examples of the aluminum hydride complex compound include lithium aluminum hydride, lithium diethoxyaluminum hydride, lithium tri-tert-butoxyaluminum hydride, diisobutylaluminum hydride, sodium bis(2-methoxyethoxy)aluminum hydride and the like.

When a borate coexists in the reduction of 6-hydroxy-2-naphthalenecarboxylic acid of the formula (II) with a borane, by-products such as a dimer and a trimer can be reduced.

Although the type of the borate used in combination is not particularly limited, the compounds represented by the following general formula (III), (IV), (V) or (VI) are preferably used.

In the following general formula (III): 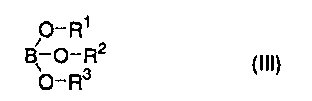 (wherein, R¹, R² and R³ independently represent an alkyl group, an aralkyl group, a hetero atom-containing alkyl group, or an aryl group), the alkyl group may be linear, branched, cyclic, or a combination thereof, having 1 to 20 carbon atoms, preferably 1 to 16 carbon atoms, and may be substituted. More specifically, examples include methyl group, ethyl group, 2,2,2-trifluoroethyl group, propyl group, butyl group, hexyl group, octyl group, decyl group, hexadecyl group, cyclohexyl group, 2-cyclohexylhexyl group and the like.

The aralkyl group has 7 to 20, preferably 7 to 16 carbon atoms, and the aryl moiety may be substituted. More specifically, examples include benzyl group, 2-phenylcyclohexyl group and the like.

The hetero atom-containing alkyl group has 1 to 20 carbon atoms, preferably 1 to 16 carbon atoms, and contains one or more hetero atoms such as oxygen atom, nitrogen atom, and sulfur atom. More specifically, examples include 2-methoxyethyl group, 2-dimethylaminoethyl group and the like.

The aryl group has 6 to 20, preferably 6-16 carbon atoms and may be substituted. More specifically, examples include phenyl group, tolyl group, 2,6-di-t-butylphenyl group, chlorophenyl group and the like.

Specific examples of the compound represented by the aforementioned general . formula (III) include trimethyl borate, triethyl borate, tris(2,2,2-trifluoroethyl) borate, tripropyl borate, triisopropyl borate, tributyl borate, tri-t-butyl borate, tri-n-amyl borate, trihexyl borate, tricyclohexyl borate, tris(1-isopropyl-2-methylpropyl) borate, tris(3,3,5-trimethylhexyl) borate, tris(1-isobutyl-3-methylbutyl) borate, tris(decyl) borate, tri-n-hexadecyl borate, tri-n-octadecyl borate, tri-n-octyl borate, tri-2-octyl borate, tri-DL-menthyl borate, tri-2-phenylcyclohexyl borate, triamylmethyl borate, 2,6-di-t-butylphenyl dibutyl borate, tris(2-ethylhexyl) borate, tris(2-cyclohexylcyclohexyl) borate, tribenzyl borate, dimethyl monoethyl borate, dimethyl monophenyl borate, monomethyl diethyl borate, triphenyl borate, tris(4-chlorophenyl) borate, tris(2-methylphenyl) borate and the like.

In the following general formula (IV): 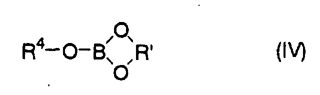 (wherein, R⁴ represents an alkyl group, an aralkyl group, a hetero atom-containing alkyl group or an aryl group, and R' represents an alkylene group which may be substituted), the alkyl group, having 1 to 16, preferably 1 to 12 carbon atoms, may be linear, branched, cyclic, or a combination thereof. More specifically, examples include methyl group, isopropyl group, n-butyl group and the like.

The aralkyl group has 7 to 20, preferably 7 to 16 carbon atoms, and the aryl moiety may be substituted. More specifically, examples include benzyl group, 2-phenylcyclohexyl group and the like.

The hetero atom-containing alkyl group has 1 to 16, preferably 1 to 12 carbon atoms, and the hetero atom may preferably be one or more of hetero atoms selected from the group consisting of oxygen atom, nitrogen atom, and boron atom. Specific examples include dimethylaminoethyl group, 2,4-dimethyl-4-(4,4,6-trimethyl-1,3,2-dioxaborinano)pentyl group and the like.

The aryl group has 6 to 20, preferably 6 to 16 carbon atoms and may be substituted. More specifically, examples include phenyl group, tolyl group, 2,6-di-t-butylphenyl group, chlorophenyl group and the like.

The alkylene group has 2 to 16, preferably 2 to 12 carbon atoms and may be substituted. Specific examples thereof include ethylene group, 1,1,2,2-tetraethylene group, 1-methyltrimethylene group, l,1,3-trimethyltrimethylene group and the like.

Specific examples of the compound represented by the aforementioned general formula (IV) include 2-(2-dimethylaminoethoxy)-4-methyl-1,3,2-dioxaborinane, 2,2'-(2-methyl-2,4-pentanediyldioxy)bis(4,4,6-trimethyl-1,3,2-dioxaborinane, 2-butoxy-4,4,6-trimethyl-1,3,2-dioxaborinane, 2-methoxy-1,3,2-dioxaborolane, 2-isopropoxy-4,4,5,5-tetramethyl-1,3,2-dioxaborolane and the like.

In the following general formula (V): 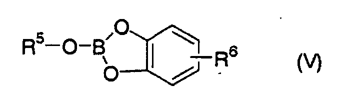 (wherein, R⁵ and R⁶ independently represent an alkyl group, an aralkyl group, a hetero atom-containing alkyl group, or an aryl group), the alkyl group, having 1 to 16, preferably 1 to 12 carbon atoms, may be linear, branched, cyclic, or a combination thereof. Specific examples thereof include methyl group, ethyl group and the like.

The aralkyl group has 7 to 16, preferably 7 to 12 carbon atoms, and the aryl moiety may be substituted.

The hetero atom-containing alkyl group has 1 to 16, preferably 1 to 12 carbon atoms, and contains one or more hetero atoms such as oxygen atom, nitrogen atom and sulfur atom. More specifically, examples include 2-methoxyethyl group, 2-dimethylaminoethyl group and the like.

The aryl group has 6 to 20, preferably 6 to 16 carbon atoms and may be substituted. More specifically, examples include phenyl group, tolyl group and the like.

Specific examples of the compound represented by the aforementioned general formula (V) include 2-methoxybenzo[d]-1,3,2-dioxaborolane, 2-ethoxybenzo[d]-1,3,2-dioxaborolane, 2-phenoxybenzo[d]-1,3,2-dioxaborolane and the like.

In the following general formula (VI): 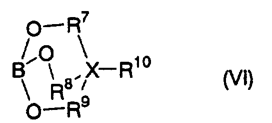 (wherein, R⁷, R⁸ and R⁹ independently represent an alkylene group which may be substituted or an alkyleneoxy group which may be substituted, R¹⁰ represents a hydrogen atom or an alkyl group, X represents a carbon atom, a nitrogen atom or a boron atom, and a, b, c and d each independently represent 0 or 1), the alkylene group which may be substituted has 1 to 16, preferably 1 to 12 carbon atoms, and specific examples thereof include methylene group, ethylene group and the like.

The alkyleneoxy group which may be substituted has 1 to 16, preferably 1 to 12 carbon atoms, and specific examples thereof include ethyleneoxy group, 1,3,3-trimethyl-trimethyleneoxy group and the like.

The alkyl group, having 1 to 16, preferably 1 to 12 carbon atoms, may be linear, branched, cyclic, or a combination thereof. More specifically, examples include methyl group, ethyl group, isopropyl group, n-butyl group and the like.

Specific examples of the compound represented by the aforementioned general formula (VI) include borates such as triethanolamine borate, 2-methyl-2,4-pentanediol diborate, 1-bora-2,6,7-trioxabicyclo[2.2.1]heptane and 1,6-dibora-2,5,7,10,11,14-hexaoxabicyclo[4.4.4]tetradecane.

Two or more kinds of these borate may be used in combination.

Among the compounds represented by these general formulas (III) to (VI), trimethyl borate, triethyl borate and the like are preferred mainly from an economical standpoint.

Commercially available products, per se, can be used as the aforementioned borates, or they can be easily prepared by reacting, with an alcohol, a trihalogenated boron compound disclosed in Lappert, M.F., Chem. Review, 56, 959 (1956), diboron trioxide, boric acid, sodium pyroboric acid ester, or borane together with a borohydride compound as raw materials. As the alcohol, any alcohols can be used without limitations by their alcoholic order, hydric valence and absence or presence of a substituent. Methanol, ethanol and the like are preferred mainly from an economical viewpoint.

The borate is used generally in 0.01 to 20 mole, preferably 0.05 to 10 mole based on 6-hydroxy-2-naphthalenecarboxylic acid.

Examples of the method for addition of the borate include a method of adding the borate to a solution of 6-hydroxy-2-naphthalenecarboxylic acid dissolved in a suitable solvent and adding dropwise the prepared mixed solution to a solution of, for example, borane/tetrahydrofuran complex in tetrahydrofuran; a method of adding the borate to a solution of borane/tetrahydrofuran complex in tetrahydrofuran and mixing the mixture, and then adding dropwise a solution of 6-hydroxy-2-naphthalenecarboxylic acid dissolved in a solvent to the mixed solution and the like. The former method is preferred because it provides 6-hydroxy-2-naphthylcarbinol with a higher purity.

For the aforementioned reaction, a borane and 6-hydroxy-2-naphthalenecarboxylic acid are generally reacted in the presence of a solvent and a borate. The type of the solvent is not particularly limited so long as the solvent is inert in the reaction. Examples include ether solvents such as diethyl ether, tetrahydrofuran, dioxane, digrim, trigrim, t-butyl methyl ether and 1,3-dioxolane; hydrocarbon solvents such as benzene, toluene and xylene; halogenated solvents such as chlorobenzene, 1,2-dichloroethane, chloroform and carbon tetrachloride and the like. The solvent is used in 1 to 50 times, preferably 2 to 20 times weight amount based on 6-hydroxy-2-naphthalenecarboxylic acid.

The reaction temperature is usually -30 to 150°C, preferably -20 to 100°C. Although the reaction time may vary depending on the types of the borane and the borate to be used, amounts thereof and the like, it is usually about 0.5 to 10 hours.

When the borane is prepared beforehand and used for the reaction, the reaction can be usually performed by adding 6-hydroxy-2-naphthalenecarboxylic acid raw material or its mixture with a solvent (a raw material solution) to a borane preparation solution. The solvent of the raw material solution may be different from that used for the preparation of borane solution. Examples of the solvents for the preparation of borane and the raw material solution include those solvents mentioned above.

A solution of borane preparation, per se, in which a borohydride compound and dimethyl sulfate or boron trifluoride are reacted may be used. The solution may also be used after a monomethyl sulfate salt or a tetrafluoroborate salt deposited in the system is removed by filtration.

After the reducing agent remaining in the reaction mixture is inactivated by addition of a mineral acid such as hydrochloric acid, water or methanol or the like and the reaction mixture was neutralized with sodium hydroxide or the like, the 6-hydroxy-2-naphthalenecarbinol produced by the method described above can be separated by an ordinarily used method such as crystallization, extraction, distillation and column chromatography.

The method for oxidizing 6-hydroxy-2-naphthylcarbinol of the formula (I) to produce 6-hydroxy-2-naphthaldehyde will be explained.

An oxidizing agent used in the reaction is not particularly limited so long as the agent enables satisfactory production of 6-hydroxy-2-naphthaldehyde. For example, manganese dioxide may preferably used. Manganese dioxide can easily be produced by electrolysis of manganese sulfate solution, heating of manganese oxide in an oxygen flow, thermolysis of manganese nitrate and the like. Mass-produced commercial products for use in dry cells, per se, may be used, and activated manganese dioxide can also be used. As the method for producing activated manganese dioxide, any method may be suitably selected from ordinarily used methods, which are known per se, and used depending on a type of a substrate, reaction temperature, a solvent or reaction time, for example, 1) the method of preparation from potassium permanganate, manganese sulfate and sodium hydroxide, 2) the method of pyrolysis of a manganese(II) salt of oxalic acid or carbonic acid at 250°C, 3) the method of treatment of manganese dioxide with nitric acid, 4) the method of dehydration of manganese dioxide by azeotropy with a solvent and the like. Commercially available activated manganese dioxide may also be used.

Manganese dioxide is used in an amount of usually 0.1 to 50 moles, preferably 1 to 30 moles, more preferably 2 to 30 moles, per mole of 6-hydroxy-2-naphthylcarbinol.

If necessary, a solvent that does not react with 6-hydroxy-2-naphthylcarbinol and manganese dioxide can also be used. Specific example thereof include hydrocarbons such as hexane, benzene and toluene; monoalcohols such as methanol, ethanol, propanol and butanol; diols such as ethylene glycol, propylene glycol, butanediol and hexylene glycol; ethers such as diethyl ether, tetrahydrofuran and dioxane; ketones such as acetone, methyl ethyl ketone, diethyl ketone, methyl propyl ketone, dipropyl ketone and methyl isobutyl ketone; esters such as methyl acetate, ethyl acetate, butyrolactone and tricaprilin; amides such as dimethylformamide, dimethylacetamide and methylpyrrolidinone; dimethylimidazolidinone, tetramethylurea and the like. Among them, hydrocarbons, ketones, ethers and amides are preferred, and ketones and amides are more preferred from viewpoints of a reaction acceleration effect and an effect of suppressing by products. An amount of the solvent to be used is not particularly limited, and the solvent may be used in an amount that allows sufficient advance of the reaction.

Although the reaction sufficiently proceeds even at a temperature below room temperature, the reaction may usually be conducted at a temperature equal to or higher than room temperature to accelerate a reaction rate. The reaction temperature is usually in the range of -10 to 200°C, preferably in the range of 0 to 100°C. The reaction may be conducted under air, or may be conducted under an atmosphere of inert gas such as nitrogen and argon. The reaction time may vary depending on the reaction temperature, and is usually in the range of 0.1 to 200 hours, preferably in the range of 0.5 to 80 hours. After manganese dioxide remaining in the reaction mixture is removed by filtration, the 6-hydroxy-2-naphthaldehyde produced by the method described above can be isolated by ordinarily used methods such as crystallization, extraction and column chromatography.

Further, as an alternative method for oxidizing 6-hydroxy-2-naphthylcarbinol of the formula (I) to produce 6-hydroxy-2-naphthaldehyde, a method is provided in which the oxidation is performed with oxygen by using a catalyst system containing a nitroxide together with a metal compound having oxidation-reduction ability or a metal complex compound having oxidation-reduction ability.

As the nitroxide, a stable free radical nitroxide is preferred. The term "stable free radical nitroxide" used herein means that the nitroxide can be prepared by an ordinary chemical reaction and can be statically detected by an usual spectroscopic method. In general, such a stable free radical nitroxide has a half-life of at least one year.

The stable free radical nitroxide is produced by oxidation of a cyclic secondary amine that does not contain hydrogen in a substituent on a carbon atom adjacent to N or an equivalent hydroxylamine by using a peroxide or the like.

Examples of the stable free radical nitroxide include, for example, compounds represented by the following general formula (VII) and (VIII).

In the compounds represented by the following general formula (VII): 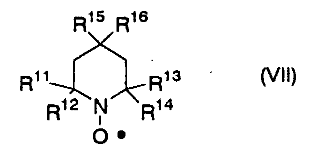 (wherein, R¹¹, R¹², R¹³ and R¹⁴ independently represent an alkyl group, an aryl group or a hetero atom-substituted alkyl group, and R¹⁵ and R¹⁶ independently represent a hydrogen atom, an alkyl group, an aryl group, or a hetero atom-containing substituent), the alkyl group, having 1 to 15, preferably 1 to 8 carbon atoms, may be linear, branched, cyclic, or a combination thereof. Specific examples include methyl group, ethyl group, n-propyl group, i-propyl group, n-butyl group, i-butyl group, t-butyl group, cyclohexyl group and the like, and methyl group is preferred among them.

The aryl group has 6 to 20, preferably 6 to 12 carbon atoms and may be substituted. Specific examples thereof include phenyl group, tolyl group, naphthyl group and the like.

The hetero atom-substituted alkyl group means an alkyl group having 1 to 20 carbon atoms, preferably 1 to 12 carbon atoms, and substituted with one or more functional groups containing one or more of oxygen atom, nitrogen atom, sulfur atom and the like. Specific examples thereof include, for example, hydroxymethyl group, hydroxyethyl group, methoxymethyl group, methoxyethyl group, aminomethyl group, aminoethyl group and the like.

Specific examples of the hetero atom-containing substituent include hydroxyl group; an alkoxycarbonyl group such as methoxycarbonyl group and ethoxycarbonyl group; carbamoyl group; cyano group; oxo group; an alkoxy group such as methoxy group, ethoxy group, isopropoxy group and t-butoxy group; an acyloxy group such as acetoxy group and benzoyloxy group; an alkylamino group such as methylamino group and ethylamino group; an acylamino groups such as acetylamino group and benzoylamino group; sulfate group (-O-SO₃H); group of -N⁺Me₃Cl⁻ and the like.

As for R¹⁵ and R¹⁶, it is preferred that the both of R¹⁵ and R¹⁶ are hydrogen atoms, one of R¹⁵ and R¹⁶ is a hydrogen atom and the other represents hydroxyl group, an alkoxy group, or a sulfate group, or R¹⁵ and R¹⁶ bind to each other to form oxo group. Among them, it is more preferred that the both of R¹⁵ and R¹⁶ are hydrogen atoms, or one of R¹⁵ and R¹⁶ is a hydrogen atom and the other represents hydroxyl group.

Specific examples of the compound represented by the aforementioned general formula (VII) include: 2,2,6,6-tetramethylpiperidine-1-oxyl, 4-hydroxy-2,2,6,6-tetramethylpiperidine-1-oxyl, 4-methoxy-2,2,6,6-tetramethylpiperidine-1-oxyl, 4-ethoxy-2,2,6,6-tetramethylpiperidine-1-oxyl, 4-isopropoxy-2,2,6,6-tetramethylpiperidine-1-oxyl, 4-t-butoxy-2,2,6,6-tetramethylpiperidine-1-oxyl, 4-acetoxy-2,2,6,6-tetramethylpiperidine-1-oxyl, 4-benzoyloxy-2,2,6,6-tetramethylpiperidine-1-oxyl, 4-cyano-2,2,6,6-tetramethylpiperidine-1-oxyl, 4-ethoxycarbonyl-2,2,6,6-tetramethylpiperidine-1-oxyl, 4-ethylamino-2,2,6,6-tetramethylpiperidine-1-oxyl, 4-carbamoyl-2,2,6,6-tetramethylpiperidine-1-oxyl, 4-benzoylamino-2,2,6,6-tetramethylpiperidine-1-oxyl, 4-oxo-2,2,6,6-tetramethylpiperidine-1-oxyl, 2,2,6,6-tetramethylpiperidine-1-oxyl-4-sulfate, 4-ethoxycarbonyl-2,2,6,6-tetramethylpiperidine-1-oxyl, 4-methyl-2,2,6,6-tetramethylpiperidine-1-oxyl, 4-ethyl-2,2,6,6-tetramethylpiperidine-1-oxyl and the like.

In the compound represented by the following general formula (VIII): 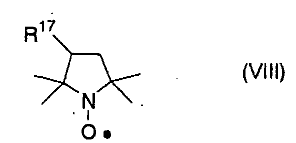 (wherein, R¹⁷ represents a hydrogen atom, an alkyl group, an alkoxycarbonyl group, a cyano group, a carbamoyl group, an alkoxy group or an acyloxy group), the alkyl group has the same meaning as that defined in the aforementioned general formula (VII). The alkoxycarbonyl group has 2 to 16, preferably 2 to 10 carbon atoms, and specific examples thereof include methoxycarbonyl group, ethoxycarbonyl group and the like.

The alkoxy group preferably has 1 to 10 carbon atoms, and examples thereof include methoxy group, ethoxy group, isopropoxy group and the like.

The acyloxy group preferably has 1 to 10 carbon atoms, and specific examples thereof include acetoxy group, benzoyloxy group and the like.

Examples of the compound of the aforementioned general formula (VIII) include, for example, 2,2,5,5-tetramethylpyrrolidine-1-oxyl, 3-methyl-2,2,5,5-tetramethylpyrrolidine-1-oxyl, 3-methoxycarbonyl-2,2,5,5-tetramethylpyrrolidine-1-oxyl, 3-cyano-2,2,5,5-tetramethylpyrrolidine-1-oxyl, 3-carbamoyl-2,2,5,5-tetramethylpyrrolidine-1-oxyl, 3-methoxy-2,2,5,5-tetramethylpyrrolidine-1-oxyl, 3-ethoxy-2,2,5,5-tetramethylpyrrolidine-1-oxyl, 3-isopropoxy-2,2,5,5-tetramethylpyrrolidine-1-oxyl, 3-t-butoxy-2,2,5,5-tetramethylpyrrolidine-1-oxyl, 3-acetoxy-2,2,5,5-tetramethylpyrrolidine-1-oxyl, 3-benzoyloxy-2,2,5,5-tetramethylpyrrolidine-1-oxyl and the like.

Among the compounds represented by the aforementioned general formula (VII) or (VIII), 2,2,6,6-tetramethylpiperidine-1-oxyl and 4-hydroxy-2,2,6,6-tetramethylpiperidine-1-oxyl can be easily obtained as industrial products and preferred.

Besides the compounds represented by the aforementioned general formula (VII) or (VIII), stable nitroxy radicals can be used such as those described in, for example, 1) de Nooy, A.E.J., Besemer, A.C., and van Bekkum, H., Synthesis, 1153 (1996), 2) Keana, J.F.W., Chem. Review, 78, 37 (1978), 3) Rozantsev, E.G., "Free Nitroxyl Radicals", Plenum Press: New York-London, 1970, 4) Rozantsev, E.G., Sholle, V.D., Synthesis, 190 (1971) and the like. These radicals include cyclic and bicyclic nitroxy radicals and polymer-like radicals in which multiple nitroxy radicals are linked together. The aforementioned nitroxides can be used alone or in combination.

Further, a cyclic secondary amine that does not contain hydrogen in a substituent on a carbon atom adjacent to N or a hydroxylamine can be oxidized in situ, or a stable free radical nitroxide may be oxidized with a hypochlorite or the like and used as an oxammonium salt.

In the present invention, the amount of the stable free radical nitroxide is 0.001 to 2 molar equivalences, preferably 0.01 to 0.5 molar equivalences based on the hydroxynaphthalenemethanol as a raw material.

Examples of a metal of the metal compound having oxidation-reduction ability and the metal complex compound having oxidation-reduction ability include, for example, copper, iron, antimony, bismuth, tin, zinc, nickel, palladium, platinum, cobalt, ruthenium, osmium, manganese, molybdenum, tungsten, vanadium, titanium, zirconium, hafnium and the like. Among them, copper and iron are preferred.

Example of copper compounds and copper complex compounds include, for example, copper compounds such as copper(I) or (II) fluoride, copper chloride, copper bromide, copper iodide, copper nitrate, copper cyanide, Li₂CuCl₃, copper perchlorate, copper hydroxide, copper sulfate, copper phosphate, copper acetate, copper tetrafluoroborate and Li₂CuCl₄, and copper complex compounds such as tetrakis(acetonitrile)copper(I) hexafluorophosphate, methyl-bis(triphenylphosphene)copper(I), methyl(2,2'-bipyridine)copper(I) and tetrachlorotetrakis(triphenylphosphine)tetracopper(I), and among them, copper chloride and copper bromide are preferred.

Examples of the iron compounds and the iron complex compounds include, for example, iron compounds such as iron(II) or iron(III) chloride, iron bromide, iron iodide, iron hydroxide, iron sulfate, iron acetate, iron nitrate, and iron phosphate, and iron complex compounds such as dichloro(triphenylphosphine)iron, and among them, iron chloride is preferred.

An amount of the aforementioned metal compound or the metal complex compound according to the present invention may be 0.001 to 2 molar equivalents, preferably 0.01 to 0.5 molar equivalents based on the 6-hydroxy-2-naphthylcarbinol.

Further, as the oxygen used in the present invention, oxygen gas diluted with inert gas such as nitrogen and argon, air or the like can be used. For introduction of the oxygen into the reaction system, in general, the reaction may be performed under an atmosphere of an oxygen-containing gas such as air, or an oxygen-containing gas may be bubbled into the reaction system using a pump or the like.

In the reaction of the present invention, if required, a solvent can be used which does not react with 6-hydroxy-2-naphthylcarbinol as the raw material and 6-hydroxy-2-naphthaldehyde as the target product, and is not oxidized under the reaction condition.

Specific examples of such solvents include aliphatic hydrocarbons such as hexane and cyelohexane; aromatic hydrocarbons such as benzene, toluene, chlorobenzene, dichlorobenzene and nitrobenzene; ethers such as ethyl ether, tetrahydrofuran and dioxane; ketones such as acetone, methyl ethyl ketone, diethyl ketone, methyl propyl ketone, dipropyl ketone and methyl isobutyl ketone; esters such as methyl acetate, ethyl acetate, butyrolactone and tricaprilin; amides such as dimethylformamide, dimethylacetamide and methylpyrrolidinone; ureas such as dimethylimidazolidinone and tetramethylurea and the like. Among them, aliphatic and aromatic hydrocarbons, ketones, ethers and amides are preferred, and amides are more preferred from view points of a reaction acceleration effect and an effect of suppressing byproducts. These solvents may be used in combination, and an amount thereof is not particularly limited.

The reaction is usually performed by charging 6-hydroxy-2-naphthylcarbinol as the raw material and the catalyst, and a solvent, if necessary, into a reaction vessel, and bubbling air in the mixture with stirring. The reaction temperature is usually in the range of -10 to 200°C, preferably in the range of 0 to 100°C. In order to increase the reaction rate, the reaction is usually performed at a temperature not lower than room temperature. Although the reaction time depends on the type of a catalyst, a solvent and reaction temperature, it is usually in the range of 0.1 to 200 hours, preferably in the range of 0.5 to 80 hours. The reaction pressure may usually be ordinary pressure. If desired, the reaction can be performed under positive or reduced pressure.

After the remaining free radical nitroxide and metal compound are removed, 6-hydroxy-2-naphthaldehyde produced by the aforementioned method can be isolated by known methods such as a crystallization, extraction and column chromatography.

The method for producing 5-[6-substituted benzyloxy-2-naphthyl]-methyl-2,4-thiazolidinedione represented by the general formula (X) from 6-hydroxy-2-naphthaldehyde will be explained with reference to the following Reaction Formula A.

### <Reaction Formula A>

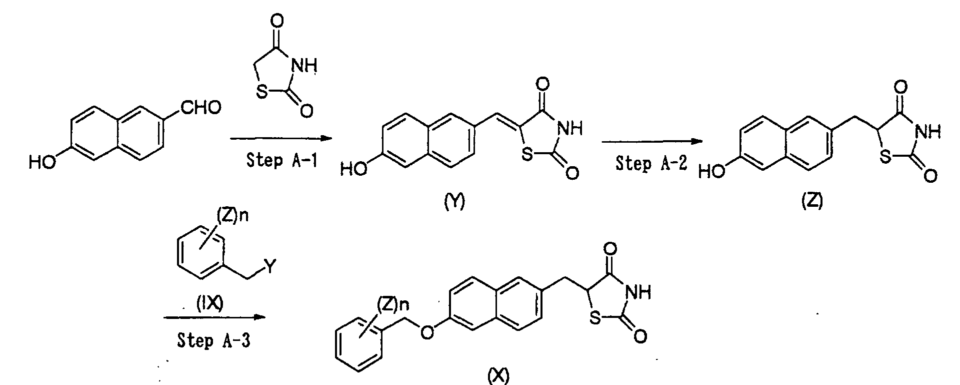

(In the formula, Y represents a halogen atom, an alkylsulfonyloxy group, or an arylsulfonyloxy group, Z represents a halogen atom and n represents an integer of 1 to 5.)

### [Step A-1]

6-Hydroxy-2-naphthaldehyde without protection of its hydroxyl group can be used for the reaction with 2,4-thiazolidinedione. For example, dehydration condensation reaction of 6-hydroxy-2-naphthaldehyde and 2,4-thiazolidinedione is carried out in the presence of a base to produce 5-(6-hydroxy-2-naphthyl)methylene-2,4-thiazolidinedione. As the base, an inorganic base, for example, an amine such as triethylamine, pyridine, piperidine, pyrrolidine, N-methylpyrrolidine and N-methylmorpholine, sodium hydroxide, potassium hydroxide, sodium carbonate, potassium carbonate, sodium acetate, potassium acetate and the like may be used, and piperidine and pyrrolidine are preferred among them. Although a solvent is not essential, for example, an alcohol such as methanol, ethanol, 1-propanol, 2-propanol and 2-methoxy ethanol, dimethyl sulfoxide (DMSO), N,N-dimethylformamide (DMF) and the like may be used as the solvent, and 2-methoxyethanol, 2-propanol, DMF and DMSO are preferred among them. These solvents may be used as a mixture. An amount of the 2,4-thiazolidinedione may be 0.8 to 10 mole, preferably 1.0 to 2.0 mole based on the 6-hydroxy-2-naphthaldehyde. The reaction temperature is 20 to 150°C, preferably 50 to 120°C. Although the reaction time may vary depending on the reaction conditions, it is usually 1 hour or more, preferably 1 to 10 hours.

### [Step A-2]

The reaction of reducing the 5-(6-hydroxy-2-naphthyl)methylene-2,4-thiazolidinedione to produce the 5-(6-hydroxy-2-naphthyl)methyl-2,4-thiazolidinedione is performed, for example, under a hydrogen atmosphere or in the presence of cyclohexene. When the reaction is performed under a hydrogen atmosphere, hydrogen gas pressure is from ordinary pressure to 20 Mpa, preferably 0.2 to 8 Mpa. The catalyst is not particularly limited, and an ordinarily used hydrogenation catalyst can be used. For example, transition metal catalysts such as those comprising palladium hydroxide, palladium/carbon, palladium/black, platinum oxide and platinum/carbon and rhodium may be used. The amount thereof is 0.01 to 1 mole, preferably 0.1 to 0.3 mole based on the 5-(6-hydroxy-2-naphthyl)methylene-2,4-thiazolidinedione. As the reaction solvent, for example, alcohols such as methanol, ethanol, 1-propanol, 2-propanol and 2-methoxyethanol; ethers such as dioxane, dimethoxymethane and tetrahydrofuran (THF); ethyl acetate; acetic acid; dimethylformamide; N-methylpyrolidone and the like may be used. The reaction temperature is usually in the range of 0 to 150°C, preferably in the range of 50 to 100°C.

### [Step A-3]

The 5-(6-hydroxy-2-naphthyl)methyl-2,4-thiazolidinedione and a compound of the following general formula (IX): 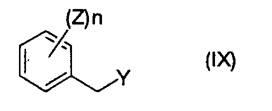 (wherein, Y, Z and n have the same meanings as defined above) can be reacted to produce 5-[6-substituted benzyloxy-2-naphthyl]-methyl-2,4-thiazolidinedione of the general formula (X).

The halogen atom mentioned in the aforementioned general formula (IX) may be a fluorine atom, chlorine atom, bromine atom, iodine atom or the like.

The alkylsulfonyloxy group contains an alkyl group that has 1 to 10 carbon atoms and may be substituted, and specific examples thereof include methylsulfonyloxy group and the like.

The arylsulfonyloxy group contains an aryl group that has 6 to 12 carbon atoms and may be substituted, and specific examples thereof include phenylsulfonyloxy group, p-tolylsulfonyloxy group and the like.

In this reaction, an amount of the compound of the general formula (IX) may be 0.8 to 3 mole, preferably 1.0 to 1.5 mole based on the 5-(6-hydroxy-2-naphthyl)methyl-2,4-thiazolidinedione. The reaction is preferably performed in the presence of a base. For example, sodium hydride, potassium hydride, sodium carbonate, potassium carbonate or the like is used as the base, and sodium hydride and potassium hydride are preferred among them. The base is used in an amount of 0.5 to 5 mole, preferably 1.0 to 3.0 mole based on the 5-(6-hydroxy-2-naphthyl)methyl-2,4-thiazolidinedione in terms of molar amount. As the solvent, for example, THF, dioxane, diethyl ether, DMF, DMSO, N-methylpyrolidone or the like may be used, and DMF, DMSO and N-methylpyrolidone are preferred among them. These solvents may be used as a mixture. The reaction temperature is usually in the range of 0 to 100°C, preferably in the range of 10 to 40°C. Although the reaction time may vary depending on the reaction conditions, it is usually 1 hour or more, preferably 3 to 10 hours.

### Examples

The present invention will be explained more specifically with reference to the following examples. However, the scope of the present invention is not limited to these examples.

### Example 1

Under a nitrogen atmosphere, 3.87 g (100 mmol) of sodium borohydride (purity: 98%) and 60 mL of anhydrous tetrahydrofuran were introduced into a 300-mL four-neck flask equipped with a dropping funnel, a gas inlet pipe, a condenser, and a thermometer and stirred. The mixture was added dropwise with 12.65 g (100 mmol) of dimethyl sulfate at 0°C over 5 minutes and stirred for 2 hours with ice cooling and further stirred at room temperature for 3 hours and the stop of gas generation was observed. The resulting borane/tetrahydrofuran complex solution in tetrahydrofuran was maintained at around 0°C, and the supernatant was filtered through a glass filter under a nitrogen atmosphere to separate sodium monomethylsulfate salt generated in the system. The layer of the salt was further washed with 40 mL of anhydrous tetrahydrofuran, combined with about 1 M purified borane/tetrahydrofuran complex solution in tetrahydrofuran and then transferred to another 500-mL four-neck flask. A solution of 9.41 g (50 mmol) of 6-hydroxy-2-naphthalenecarboxylic acid and 20.13 g of trimethyl borate in anhydrous tetrahydrofuran (50 mL) was added dropwise to the prepared borane/tetrahydrofuran complex solution at room temperature over 25 minutes by using a dropping funnel. This reaction mixture was stirred at the same temperature for 3 hours. After the reaction was completed, the reaction mixture was cooled, added with 100 mL of water with ice cooling and vigorously stirred at room temperature for 30 minutes. After the tetrahydrofuran was evaporated under reduced pressure, white crystals were deposited. The resulting white crystals were washed with water (200 mL) by filtration and dried under reduced pressure at 80°C for 8 hours to obtain 8.46 g of 6-hydroxy-2-naphthylcarbinol as white crystals.

The composition of the resulting white crystals was analyzed by liquid chromatography using the internal standard method. As a result, it was found that the conversion of the 6-hydroxy-2-naphthalenecarboxylic acid was 99.93% and the purity of the 6-hydroxy-2-naphthylcarbinol was 99.21% (48.57 mmol, yield: 97.1%). It was also found that there were 0.37% of 6-hydroxy-2-naphthaldehyde as a by-product and 0.35% in total of other by-products. Physicochemical properties of this 6-hydroxy-2-naphthylcarbinol are shown below.
Melting point: 181-183°C

| Elemental analysis: as C₁₁H₁₀O₂ (174.20) | | |
|---|---|---|
| Calcd | C, 75.84; | H, 5.79 |
| Found | C, 75.89; | H, 5.83 |

MS (m/e): 174 (M⁺, 100%), 157, 145, 127, 115
IR (KBr): 3417, 3183, 2864, 1607, 1214, 1171, 1009, 999, 903, 871 cm⁻¹
¹H-NMR (300 MHz, CDCl₃ + DMSO-d₆, δ ppm): 9.08 (1H, brs), 7.70-7.58 (3H, m), 7.40 (1H, d), 7.10 (2H, q), 4.72 (2H, s), 2.91-(1H, brs)
¹³C-NMR (300 MHz, CDCl₃ + DMSO-d₆, δ ppm): 63.28, 108.10, 117.79, 124.11, 124.92, 125.23, 127.03, 128.20, 133.15, 135.57, 154.23

### Example 2

Under a nitrogen atmosphere, 100 mL of about 1 M borane/tetrahydrofuran complex solution in tetrahydrofuran obtained by the same procedure as in Example 1 was introduced into a 500 mL four-neck flask equipped with a dropping funnel, a gas inlet pipe, a condenser, and a thermometer and added dropwise with a solution of 12.58 g (66.85 mmol) of 6-hydroxy-2-naphthalenecarboxylic acid and 6.95 g of trimethyl borate in anhydrous tetrahydrofuran (70 mL) at room temperature over 30 minutes by using a dropping funnel. This reaction mixture was stirred at the same temperature for 3 hours. After the reaction was completed, the reaction mixture was cooled, added with 150 mL of water with ice cooling and vigorously stirred at room temperature for 30 minutes. After the tetrahydrofuran was evaporated under reduced pressure, white crystals were deposited. The resulting white crystals were washed with water (200 mL) by filtration and dried under reduced pressure at 80°C for 8 hours to obtain 11.28 g of 6-hydroxy-2-naphthylcarbinol as white crystals.

The composition of the resulting white crystals was analyzed by liquid chromatography using the internal standard method. As a result, it was found that the conversion of the 6-hydroxy-2-naphthalenecarboxylic acid was 99.99% and the purity of the 6-hydroxy-2-naphthylcarbinol was 99.42% (64.75 mmol, yield: 96.9%). It was also found that there were 0.08% of 6-hydroxy-2-naphthaldehyde, 0.07% of 1-[(6-hydroxy(2-naphthyl))methyl]-6-(hydroxymethyl)naphthalen-2-ol, 0.10% of 6-[(6-hydroxy-2-naphthyloxy)methyl]naphthalen-2-ol, 0.05% of (6-hydroxy-2-naphthyl)carbonyl-6-hydroxynaphthalene-2-carboxylate as by products and 0.27% in total of other by products.

### Example 3

Under a nitrogen atmosphere, 100 mL of about 1 M borane/tetrahydrofuran complex solution in tetrahydrofuran obtained by the same procedure as in Example 1 was introduced into a 500-mL four-neck flask equipped with a dropping funnel, a gas inlet pipe, a condenser, and a thermometer and added dropwise with a solution of 12.60 g (66.96 mmol) of 6-hydroxy-2-naphthalenecarboxylic acid and 19.27 g triethyl borate in anhydrous tetrahydrofuran (70 mL) at room temperature over 35 minutes by using a dropping funnel. This reaction mixture was stirred at the same temperature for 5 hours. After the reaction was completed, the reaction mixture was cooled, added with 200 mL of water with ice cooling and vigorously stirred at room temperature for 30 minutes. After the tetrahydrofuran was evaporated under reduced pressure, white crystals were deposited. The resulting white crystals were washed with water (250 mL) by filtration and dried under reduced pressure at 80°C for 8 hours to obtain 11.36 g of 6-hydroxy-2-naphthylcarbinol as white crystals.

The composition of the resulting white crystals was analyzed by liquid chromatography using the internal standard method. As a result, it was found that the conversion of the 6-hydroxy-2-naphthalenecarboxylic acid was 99.92% and the purity of the 6-hydroxy-2-naphthylcarbinol was 99.18% (65.21 mmol, yield: 97.4%). It was also found that there were 0.31% of 6-hydroxy-2-naphthaldehyde, 0.06% of 1-[(6-hydroxy(2-naphthyl))methyl]-6-(hydroxymethyl)naphthalen-2-ol, 0.05% of (6-hydroxy-2-naphthyl)carbonyl-6-hydroxynaphthalene-2-carboxylate as by products and 0.32% in total of other by products.

### Example 4

Under a nitrogen atmosphere, 100 mL of about 1 M borane/tetrahydrofuran complex solution in tetrahydrofuran obtained by the same procedure as in Example 1 and 13.86 g trimethyl borate were introduced into a 500 mL four-neck flask equipped with a dropping funnel, a gas inlet pipe, a condenser and a thermometer, and added dropwise with a solution of 12.56 g (66.74 mmol) of 6-hydroxy-2-naphthalenecarboxylic acid in anhydrous tetrahydrofuran (70 mL) at room temperature over 40 minutes by using a dropping funnel. This reaction mixture was stirred at the same temperature for 4 hours. After the reaction was completed, the reaction mixture was cooled, added with 150 mL of water with ice cooling and vigorously stirred at room temperature for 30 minutes. After the tetrahydrofuran was evaporated under reduced pressure, white crystals were deposited. The resulting white crystals were washed with water (200 mL) by filtration and dried under reduced pressure at 80°C for 8 hours to obtain 11.23 g of 6-hydroxy-2-naphthylcarbinol as white crystals.

The composition of the resulting white crystals was analyzed by liquid chromatography using the internal standard method. As a result, it was found that the conversion of the 6-hydroxy-2-naphthalenecarboxylic acid was 99.95% and the purity of the 6-hydroxy-2-naphthylcarbinol was 99.26% (64.47 mmol, yield: 96.6%). It was also found that there were 0.28% of 6-hydroxy-2-naphthaldehyde, 0.05% of 1-[(6-hydroxy(2-naphthyl))methyl]-6-(hydroxymethyl)naphthalen-2-ol as by-products and 0.36% in total of other by-products.

### Example 5

Under a nitrogen atmosphere, 3.91 g (101 mmol) of sodium borohydride (purity: 98%) and 60 mL of anhydrous tetrahydrofuran were introduced into a 500 mL four-neck flask equipped with a dropping funnel, a gas inlet pipe, a condenser and a thermometer and then stirred. The mixture was added dropwise with 13.17 g (104 mmol) of dimethyl sulfate at 0°C over 5 minutes and stirred for 2 hours with ice cooling. The mixture was further added with 40 mL of anhydrous tetrahydrofuran and stirred at room temperature for 3 hours, and the stop of gas generation was observed. Subsequently, the reaction mixture was added dropwise with a solution of 12.59 g (66.90 mmol) of 6-hydroxy-2-naphthalenecarboxylic acid and 6.96 g of trimethyl borate in anhydrous tetrahydrofuran (70 mL) at room temperature over 35 minutes by using a dropping funnel. This reaction mixture was stirred at the same temperature for 2 hours. After the reaction was completed, the reaction mixture was cooled, added with 200 mL of water with ice cooling and vigorously stirred at room temperature for 30 minutes. After the tetrahydrofuran was evaporated under reduced pressure, white crystals were deposited. The resulting white crystals were washed with water (200 mL) by filtration and dried under reduced pressure at 80°C for 8 hours to obtain 11.31 g of 6-hydroxy-2-naphthylcarbinol as white crystals.

The composition of the resulting white crystals was analyzed by liquid chromatography using the internal standard method. As a result, it was found that the conversion of the 6-hydroxy-2-naphthalenecarboxylic acid was 99.95% and the purity of the 6-hydroxy-2-naphthylcarbinol was 99.30% (64.93 mmol, yield: 97.1%). It was also found that there were 0.10% of 6-hydroxy-2-naphthaldehyde, 0.04% of 1-[(6-hydroxy(2-naphthyl))methyl]-6-(hydroxymethyl)naphthalen-2-ol, 0.12% of 6-[(6-hydroxy-2-naphthyloxy)methyl]naphthalen-2-ol, 0.07% of (6-hydroxy-2-naphthyl)carbonyl-6-hydroxynaphthalene-2-carboxylate as by-products and 0.32% in total of other by products.

### Example 6

Under a nitrogen atmosphere, 200 mL of about 1 M borane/tetrahydrofuran complex solution in tetrahydrofuran obtained by the same procedure as in Example 1 was introduced into a 500 mL four-neck flask equipped with a dropping funnel, a gas inlet pipe, a condenser and a thermometer, and then added dropwise with a solution of 18.82 g (100.0 mmol) of 6-hydroxy-2-naphthalenecarboxylic acid in anhydrous tetrahydrofuran (120 mL) at room temperature over 1 hour by using a dropping funnel. This reaction mixture was stirred at the same temperature for 7 hours. After the reaction was completed, the reaction mixture was cooled, added with 200 mL of water with ice cooling and vigorously stirred at room temperature for 30 minutes. After the tetrahydrofuran was evaporated under reduced pressure, white crystals were deposited. The resulting white solid was washed with water (300 mL) by filtration and dried under reduced pressure to obtain 16.73 g of white solid.

The composition of the resulting white solid was analyzed by liquid chromatography using the internal standard method. As a result, it was found that the conversion of the 6-hydroxy-2-naphthalenecarboxylic acid was 99.44% and the purity of the 6-hydroxy-2-naphthylcarbinol was 87.60%. It was also found that there were 0.14% of 6-hydroxy-2-naphthaldehyde, 3.59% of 1-[(6-hydroxy(2-naphthyl))methyl]-6-(hydroxymethyl)naphthalen-2-ol, 3.29% of 6-[(6-hydroxy-2-naphthyloxy)methyl]naphthalen-2-ol, 1.24% of (6-hydroxy-2-naphthyl)carbonyl-6-hydroxynaphthalene-2-carboxylate as by-products and 3.58% in total of other by products.

### Example 7

Under a nitrogen atmosphere, 3.90 g (101 mmol) of sodium borohydride (purity: 98%) and 60 mL of anhydrous tetrahydrofuran were introduced into a 300-mL four-neck flask equipped with a dropping funnel, a gas inlet pipe, a condenser and a thermometer and stirred. The mixture was added dropwise with 13.21 g (105 mmol) of dimethyl sulfate at 0°C over 5 minutes and stirred for 2 hours with ice cooling. The mixture was further added with 40 mL of anhydrous tetrahydrofuran and stirred at room temperature for 3 hours, and the stop of gas generation was observed. Subsequently, the reaction mixture was added with a solution of 9.73 g (51.71 mmol) of 6-hydroxy-2-naphthalenecarboxylic acid in anhydrous tetrahydrofuran (50mL) at room temperature over 25 minutes by using a dropping funnel. The reaction mixture solidified during the addition. This reaction mixture was stirred at the same temperature for 7 hours. After the reaction was completed, the reaction mixture was cooled, added with 150 mL of water with ice cooling and vigorously stirred at room temperature for 30 minutes.

The resulting reaction mixture was analyzed by liquid chromatography using the internal standard method. As a result, it was found that the conversion of the 6-hydroxy-2-naphthalenecarboxylic acid was 68.53% and the yield of the 6-hydroxy-2-naphthylcarbinol was 26.8%. It was also found that there were 0.36% of 6-hydroxy-2-naphthaldehyde, 8.21% of 1-[(6-hydroxy(2-naphthyl))methyl]-6-(hydroxymethyl)naphthalen-2-ol, 6.18% of 6-[(6-hydroxy-2-naphthyloxy)methyl]naphthalen-2-ol, 5.66% of (6-hydroxy-2-naphthyl)carbonyl-6-hydroxynaphthalene-2-carboxylate as by products and a large number of other by products.

### Example 8

Under a nitrogen atmosphere, 5.49 g (0.134 mol) of sodium borohydride (purity: 92%) and 100 mL of anhydrous tetrahydrofuran were introduced into a 300-mL four-neck flask equipped with a dropping funnel, a gas inlet pipe and a thermometer and stirred. The mixture was added dropwise with 17.35 g (0.135 mol) of dimethyl sulfate at 20°C over 30 minutes and further stirred for 3 hours, and the stop of gas generation was observed. The generated borane/tetrahydrofuran complex was maintained at 20°C, and the supernatant was filtered through a glass filter under a nitrogen atmosphere to separate sodium monomethylsulfate salt generated in the system and then transferred to another 500-mL four-neck flask. A solution of 12.6 g (0.067 mol) of 6-hydroxy-2-naphthalenecarboxylic acid in anhydrous tetrahydrofuran (80 mL) was added dropwise to the resulting borane/tetrahydrofuran complex solution at 0°C over 40 minutes by using a dropping funnel. This reaction mixture was stirred at the same temperature for 15 minutes until generation of hydrogen was completed and then further stirred for 4 hours under a reflux condition. After the reaction was completed, the reaction mixture was cooled, added with 80 mL of water with ice cooling and vigorously stirred at room temperature for 30 minutes. After the tetrahydrofuran was evaporated under reduced pressure, the residue was extracted twice with 500 mL of diethyl ether. The resulting organic layer was washed with 150 mL of saturated aqueous sodium hydrogencarbonate and then with 150 mL of saturated brine and dried over magnesium sulfate. The diethyl ether was removed under reduced pressure to obtain 11.45 g (purity: 91.2%, yield: 89.5%) of 6-hydroxy-2-naphthylcarbinol as white solid. Physicochemical properties of this 6-hydroxy-2-naphthylcarbinol are shown below.
IR (KBr): 3417, 3183, 2864, 1607, 1214, 1171, 1009, 999, 903, 871 cm⁻¹
¹H-NMR (300 MHz, CDCl₃ + DMSO-d₆, δ ppm): 9.08 (1H, brs), 7.70-7.58 (3H, m), 7.40 (1H, d), 7.10 (2H, q), 4.72 (2H, s), 2.91 (1H, brs)
¹³C-NMR (300 MHz, CDCl₃ + DMSO-d₆, δ ppm): 63.28, 108.10, 117.79, 124.11, 124.92, 125.23, 127.03, 128.20, 133.15, 135.57, 154.23

### Example 9

A stirring bar, 1.5 g (8.61 mmol) of 6-hydroxy-2-naphthylcarbinol and 60 mL of N,N-dimethylformamide were introduced into a 200-mL flask to form homogenous mixture. This mixture was added with 15 g (147 mmol) of commercially available activated manganese dioxide (Aldrich, purity: 85%) and vigorously stirred for 12 hours, while the mixture was maintained at 20°C. The reaction mixture was analyzed by using liquid chromatography. As a result, it was found that the conversion of 6-hydroxy-2-naphthylcarbinol was 100% and the yield of 6-hydroxy-2-naphthaldehyde was 94%. Subsequently, the manganese dioxide was removed from the reaction mixture by filtration using Celite under reduced pressure and washed with 60 mL of ethyl acetate. The filtrate and the wash were combined and the solvent was removed under reduced pressure to obtain orange color solid. The resulting solid was dissolved in 100 mL of 0.1 N aqueous sodium hydroxide. After the remaining insoluble solids were removed by filtration, the solution was washed twice with 100 mL of dichloromethane. The aqueous layer was neutralized (pH = 6) by addition of diluted aqueous hydrochloric acid. The generated white solids were taken by filtration and further washed with water until the filtrate became neutral. Then, the solid was dried under reduced pressure at 80°C for 12 hours to obtain 0.83 g (4.82 mmol, yield: 56%) of 6-hydroxy-2-naphthaldehyde. Physicochemical properties of this 6-hydroxy-2-naphthaldehyde are shown below.
Melting point: 182-183°C
¹H-NMR (300 MHz, CDCl₃, δ ppm): 10.09 (1H, s), 8.27 (1H, s), 7.93 (2H, dd), 7.76 (1H, d), 7.22-7.18 (2H, m), 5.48 (1H, s)
¹³C-NMR (300 MHz, CDCl₃ + DMSO-d₆, δ ppm): 107.81, 118.28, 121.15, 125.24, 125.44, 129.80, 129.86, 133.16, 136.70, 157.07, 190.23

### Example 10

A stirring bar, 0.35 g (2.01 mmol) of 6-hydroxy-2-naphthylcarbinol and 15 mL of N,N-dimethylformamide were introduced into a 100-mL flask to form homogenous mixture. This mixture was added with 1.8 g (17.6 mmol) of commercially available activated manganese dioxide (Aldrich, purity: 85%) and vigorously stirred for 9 hours, while the mixture was maintained at 20°C. The reaction mixture was analyzed by using liquid chromatography. As a result, it was found that the conversion of the 6-hydroxy-2-naphthylcarbinol was 98.3% and the yield of the 6-hydroxy-2-naphthaldehyde was 93%.

### Example 11

A stirring bar, 0.35 g (2.01 mmol) of 6-hydroxy-2-naphthylcarbinol and 30 mL of acetone were introduced into a 100 mL flask to form homogenous mixture. This mixture was added with 3.5 g (34.22 mmol) of commercially available activated manganese dioxide (Aldrich, purity: 85%) and vigorously stirred for 12 hours, while the mixture was maintained at 20°C. The reaction mixture was analyzed by using liquid chromatography. As a result, it was found that the conversion of the 6-hydroxy-2-naphthylcarbinol was 96.5% and the yield of the 6-hydroxy-2-naphthaldehyde was 91%.

### Example 12

A stirring bar, 0.35 g (2.01 mmol) of 6-hydroxy-2-naphthylcarbinol and 20 mL of N,N-dimethylformamide were introduced into a 100 mL flask to form homogenous mixture. This mixture was added with 3.5 g (36.94 mmol) of electrolytic manganese dioxide (FMH, Tosoh, purity: 91.77%) and vigorously stirred for 16 hours, while the mixture was maintained at 20°C. The reaction mixture was analyzed by using liquid chromatography. As a result, it was found that the conversion of the 6-hydroxy-2-naphthylcarbinol was 92.3% and the yield of the 6-hydroxy-2-naphthaldehyde was 87%.

### Example 13

A stirring bar, 0.35 g (2.01 mmol) of 6-hydroxy-2-naphthylcarbinol and 20 mL of N,N-dimethylformamide were introduced into a 100-mL flask to form homogenous mixture. This mixture was added with 3.5 g (37.1 mmol) of electrolytic manganese dioxide (HMH, Tosoh, purity: 92.15%) and vigorously stirred for 32 hours, while the mixture was maintained at 20°C. The reaction mixture was analyzed by using liquid chromatography. As a result, it was found that the conversion of the 6-hydroxy-2-naphthylcarbinol was 94.5% and the yield of the 6-hydroxy-2-naphthaldehyde was 89%.

### Example 14

A stirring bar, 0.35 g (2.01 mmol) of 6-hydroxy-2-naphthylcarbinol and 20 mL of N,N-dimethylformamide were introduced into a 100 mL flask to form homogenous mixture. This mixture was added with 3.5 g (37.11 mmol) of electrolytic manganese dioxide (HH, Tosoh, purity: 92.18%) and vigorously stirred for 32 hours, while the mixture was maintained at 20°C. The reaction mixture was analyzed by using liquid chromatography. As a result, it was found that the conversion of the 6-hydroxy-2-naphthylcarbinol was 89.2% and the yield of the 6-hydroxy-2-naphthaldehyde was 84%.

### Example 15

2.56 g (25.86 mmol) of copper (I) chloride, 4.04 g (25.86 mmol) of 2,2,6,6-tetramethylpiperidine-1-oxyl, 90.1 g (0.517 mol) of 6-hydroxy-2-naphthylcarbinol and 0.6 L of N,N-dimethylformamide were introduced into a 1-L four-neck flask equipped with a stirrer, a gas bubbling pipe, a thermometer and a condenser, and then stirred at room temperature by air bubbling at a rate of 200 to 210 mL/min. After air bubbling for 9 hours, the reaction mixture was analyzed by liquid chromatography. As a result, it was found that the conversion of the 6-hydroxy-2-naphthylcarbinol was 99.6% and the yield of the 6-hydroxy-2-naphthaldehyde was 91.3%. Subsequently, the reaction mixture was cooled, slowly added dropwise with 10% aqueous hydrochloric acid (50 mL) with ice cooling and further added with water (1.8 L). The resulting crystals were separated at room temperature by filtration. These crystals were dissolved in ethyl acetate (2 L) and the resulting solution was washed twice with saturated aqueous sodium hydrogencarbonate (0.5 L), twice with aqueous saturated sodium sulfite (0.5 L) and twice with saturated brine (0.5 L) and then dried over magnesium sulfate. The ethyl acetate was removed under reduced pressure and the residue was dried under reduced pressure at 60°C for 8 hours to obtain 74.6 g (0.433 mol, yield: 83.8%, purity: 99.8%) of 6-hydroxy-2-naphthaldehyde as white yellow crystals.
Physicochemical properties of this 6-hydroxy-2-naphthaldehyde are shown below.
Melting point: 182-183°C
¹H-NMR (300 MHz, CDCl₃, δ ppm): 10.09 (1H, s), 8.27 (1H, s), 7.93 (2H, dd), 7.76 (1H, d), 7.22-7.18 (2H, m), 5.48 (1H, s)
¹³C-NMR (300 MHz, CDCl₃ + DMSO-d₆, δ ppm): 107.81, 118.28, 121.15, 125.24, 125.44, 129.80, 129.86, 133.16, 136.70, 157.07, 190.23

### Example 16

7.46 g (52.0 mmol) of copper (I) bromide, 4.04 g (25.86 mmol) of 2,2,6,6-tetramethylpiperidine-1-oxyl, 90.5 g (0.52 mol) of 6-hydroxy-2-naphthylcarbinol and 0.6 L of N,N-dimethylformamide were introduced into a 1-L four-neck flask equipped with a stirrer, a gas bubbling pipe, a thermometer and a condenser, and then stirred by bubbling with an oxygen gas diluted with an approximately equivalent volume of nitrogen at a rate of 100 to 110 mL/min with ice cooling. After the bubbling for 6 hours with ice cooling, the reaction mixture was analyzed by liquid chromatography. As a result, it was found that the conversion of the 6-hydroxy-2-naphthylcarbinol was 99.8% and the yield of the 6-hydroxy-2-naphthaldehyde was 92.3%. Subsequently, the reaction mixture was cooled, slowly added dropwise with 10% aqueous hydrochloric acid (50 mL) with ice cooling and further added with water (1.8 L). The resulting crystals were separated at room temperature by filtration. These crystals were dissolved in ethyl acetate (2 L), and the resulting solution was washed twice with saturated aqueous sodium hydrogencarbonate (0.5 L), twice with saturated aqueous sodium sulfite (0.5 L) and twice with saturated brine (0.5 L) and then dried over magnesium sulfate. The ethyl acetate was removed under reduced pressure and the residue was dried under reduced pressure at 60°C for 8 hours to obtain 70.5 g (0.409 mol, yield: 78.7%, purity: 99.6%) of 6-hydroxy-2-naphthaldehyde as white yellow crystals.

### Example 17

2.56 g (25.86 mmol) of copper (I) chloride, 4.46 g (25.89 mmol) of 4-hydroxy-2,2,6,6-tetramethylpiperidine-1-oxyl, 90.1 g (0.517 mol) of 6-hydroxy-2-naphthylcarbinol and 0.6 L of N,N-dimethylformamide were introduced into a 1-L four-neck flask equipped with a stirrer, a gas bubbling pipe, a thermometer and a condenser, and then stirred at room temperature by bubbling with an oxygen gas diluted with an approximately equivalent volume of nitrogen at a rate of 100 to 110 mL/min. After bubbling for 10 hours, the reaction mixture was analyzed by liquid chromatography.
As a result, it was found that the conversion of the 6-hydroxy-2-naphthylcarbinol was 99.5% and the yield of the 6-hydroxy-2-naphthaldehyde was 90.1%. Subsequently, the reaction mixture was cooled, slowly added dropwise with 10% aqueous hydrochloric acid (50 mL) with ice cooling and further added with water (1.8 L). The resulting crystals were separated at room temperature by filtration. These crystals were dissolved in ethyl acetate (2 L) and the resulting solution was washed twice with saturated aqueous sodium hydrogencarbonate (0.5 L), twice with saturated aqueous sodium sulfite (0.5 L) and twice with saturated brine (0.5 L) and then dried over magnesium sulfate. The ethyl acetate was removed under reduced pressure and the residue was dried under reduced pressure at 60°C for 8 hours to obtain 72.8 g (0.423 mol, yield: 81.8%, purity: 99.7%) of 6-hydroxy-2-naphthaldehyde as white yellow crystals.

### Example 18

In an amount of 3.81 g (30.05 mmol) of iron (II) chloride, 4.46 g (25.86 mmol) of 2,2,6,6-tetramethylpiperidine-1-oxyl, 90.1 g (0.517 mol) of 6-hydroxy-2-naphthylcarbinol and 0.6 L of N,N-dimethylformamide were introduced into a 1-L four-neck flask equipped with a stirrer, a gas bubbling pipe, a thermometer and a condenser, and then stirred at room temperature by bubbling with an oxygen gas diluted with an approximately equivalent amount of nitrogen at a rate of 100 to 110 mL/min. After bubbling for 10 hours, the reaction mixture was analyzed by liquid chromatography.
As a result, it was found that the conversion of the 6-hydroxy-2-naphthylcarbinol was 99.2% and the yield of the 6-hydroxy-2-naphthaldehyde was 88.5%. Subsequently, the reaction mixture was cooled, slowly added dropwise with 10% aqueous hydrochloric acid (50 mL) with ice cooling and further added with water (1.8 L). The produced crystals were separated at room temperature by filtration. These crystals were dissolved in ethyl acetate (2 L) and the resulting solution was washed twice with saturated aqueous sodium hydrogencarbonate (0.5 L), twice with saturated aqueous sodium sulfite (0.5 L) and twice with saturated brine (0.5 L) and then dried over magnesium sulfate. The ethyl acetate was removed under reduced pressure and the residue was dried under reduced pressure at 60°C for 8 hours to obtain 71.2 g (0.414 mol, yield: 80.0%, purity: 99.5%) of 6-hydroxy-2-naphthaldehyde as white yellow crystals.

### Example 19

In an amount of 1.13 g (1.18 mmol) of dichlorotris(triphenylphosphine)ruthenium complex, 0.46 g (2.94 mmol) of 2,2,6,6-tetramethylpiperidine-1-oxyl, 10.2 g (58.6 mmol) of 6-hydroxy-2-naphthylcarbinol and 100 mL of chlorobenzene were introduced into a 300-mL four-neck flask equipped with a stirrer, a gas bubbling pipe, a thermometer and a condenser and stirred by bubbling with an oxygen gas diluted with an approximately equivalent volume of nitrogen at a rate of 50 to 60 mL/min while the reaction temperature was maintained at 100°C. After bubbling for 8 hours, the reaction mixture was analyzed by liquid chromatography. As a result, it was found that the conversion of the 6-hydroxy-2-naphthylcarbinol was 92.6% and the yield of the 6-hydroxy-2-naphthaldehyde was 86.2%. Subsequently, the reaction mixture was cooled, slowly added dropwise with 10% aqueous hydrochloric acid (10 mL) with ice cooling and further added with water (60 mL). The resulting crystals were extracted with ethyl acetate (200 mL). The organic layer was washed twice with saturated aqueous sodium hydrogencarbonate (60 mL), twice with saturated aqueous sodium sulfite (60 mL) and twice with saturated brine (60 mL) and then dried over magnesium sulfate. The extract was concentrated to about 50 mL by using an evaporator and added with n-heptane (50 mL). The deposited crystals were taken by filtration and dried to obtain 7.93 g (46.06 mmol, yield: 78.6%, purity: 98.8%) of 6-hydroxy-2-naphthaldehyde as white crystals.

### Example 20 (Preparation of Compound (Y) from 6-hydroxy-2-naphthaldehyde)

6-Hydroxy-2-naphthaldehyde (1.0 g, 5.81 mmol) was dissolved in methoxyethanol (5 mL), and the solution was added with 2,4-thiazolidinedione (1.02 g, 8.71 mmol) and piperidine (0.17 mL, 1.74 mmol) and then stirred at 95°C for 5.5 hours. The reaction mixture was cooled to room temperature and then added with 33% aqueous acetic acid (16 mL). The deposited solid was collected by filtration, washed with methanol (10 mL) and dried under reduced pressure at 25°C to obtain 5-(6-hydroxy-2-naphthyl)methylene-2,4-thiazolidinedione (Compound (Y), 1.42 g, yield: 90%). Physicochemical properties of this 5-(6-hydroxy-2-naphthyl)methylene-2,4-thiazolidinedione are shown below.
NMR (DMSO-d₆), δ: 7.1-7.2 (m, 2H), 7.54 (dd, 1H, J=1.7, 8.8Hz), 7.79 (d, 1H, J=8.8Hz), 7.8-8.0 (m, 2H), 8.03 (s, 1H), 10.2 (s, 1H), 12.6 (s, 1H)
IR (KBr): 3396, 3140, 1730, 1587, 1325, 1186, 1020, 866 cm⁻¹
Melting point: > 200°C (decomposition)

### Example 21 (Production of Compound (Z) from Compound (Y))

(A) In an autoclave, the 5-(6-hydroxy-2-naphthyl)methylene-2,4-thiazolidinedione (200 mg, 0.74 mmol) obtained above was dissolved in ethyl acetate (6 mL), added with palladium hydroxide (carrying 20% of carbon, 200 mg) and stirred at 100°C for 5 hours under a hydrogen atmosphere pressurized to 6.0 kg/cm². After the reaction mixture was cooled to room temperature and the palladium catalyst was removed by filtration, the filtrate was concentrated under reduced pressure to obtain a crude product. The product was added with ethyl acetate (1.6 mL) to once form a uniform solution, and the solution was added with heptane (2 mL) to allow deposition of crystals. The crystals were washed by suspension under reflux by heating. The crystals were cooled to room temperature and taken by filtration under reduced pressure to obtain 5-(6-hydroxy-2-naphthyl)methyl-2,4-thiazolidinedione (163 mg, yield: 81%). Physicochemical properties of the resulting 5-(6-hydroxy-2-naphthyl)methyl-2,4-thiazolidinedione are shown below.
   NMR (DMSO-d₆) δ : 3.20 (dd, 1H, J=9.3, 14.3Hz), 3.48 (dd, 1H, J=4.3, 14.0Hz), 4.97 (dd, 1H, J=4.3, 9.3Hz), 7.06 (d, 1H, J=8.4Hz), 7.08 (s, 1H), 7.27 (d, 1H, J=8.5Hz), 7.60 (s, 1H), 7.62 (d, 1H, J=9.0Hz), 7.69 (d, 1H, J=9.0Hz), 9.71 (s, 1H), 12.0 (s, 1H)
   IR (KBr): 3418, 3155, 3055, 1925, 1747, 1631, 1392, 1329, 1207, 866 cm⁻¹
   Melting point: 231-233°C
(B) In an autoclave, the 5-(6-hydroxy-2-naphthyl)methylene-2,4-thiazolidinedione (400 mg, 1.48 mmol) obtained above was dissolved in ethyl acetate (12 mL), and the solution was added with palladium hydroxide (moisture content: 50%, carrying 20% of carbon, 81 mg) and stirred at 100°C for 6 hours under a hydrogen atmosphere pressurized to 50 kg/cm². The reaction mixture was cooled to room temperature, added with THF (4 mL) and the palladium catalyst was removed by filtration. Then, the filtrate was concentrated under reduced pressure to obtain a crude product. The product was purified in the same manner as in (A) to obtain 5-(6-hydroxy-2-naphthyl)methyl-2,4-thiazolidinedione (386 mg, yield: 96%).
(C) In an autoclave, the 5-(6-hydroxy-2-naphthyl)methylene-2,4-thiazolidinedione (150 g, 0.56 mmol) obtained above was dissolved in ethyl acetate (4.5 L), added with palladium hydroxide (moisture content: 50%, carrying 20% of carbon, 61 g) and stirred at 100°C for 9 hours under hydrogen atmosphere pressurized to 72-74 kg/cm². The reaction mixture was cooled to room temperature and added with THF (1.5 L), and the palladium catalyst was removed by filtration. Then, the filtrate was concentrated under reduced pressure to obtain a crude product. The product was purified in the same manner as in (A) to obtain 5-(6-hydroxy-2-naphthyl)methyl-2,4-thiazolidinedione (128 g, yield: 85%).

### Example 22 (Preparation of 5-[6-{2-fluorobenzyloxy}-2-naphthyl]-methyl-2,4-thiazolidinedione (Compound (X)) from Compound (Z))

(A) A mixture of sodium hydride (content: 60%, 1.74 g, 45.7 mmol) and DMF (206 mL) was added with the 5-(6-hydroxy-2-naphthyl)methyl-2,4-thiazolidinedione (5.00 g, 18.3 mmol) obtained above and dissolved in DMF (44 mL) with ice cooling and stirred at 10 to 15°C for 1.5 hours. Then, the mixture was added with 2-fluorobenzyl chloride (2.6 mL, 22.2 mmol) and stirred at 10 to 15°C for 1.5 hours and at 20 to 25°C for further 4 hours. The reaction mixture was added with saturated aqueous ammonium chloride (3.3 mL) to stop the reaction and further added with heptane (64 mL). The oil was washed and separated, and the DMF layer was added to saturated aqueous ammonium chloride (760 mL). The deposited solid was separated by filtration and dissolved in ethyl acetate (100 mL). The organic layer was washed with water, dried over magnesium sulfate and concentrated under reduced pressure to obtain an oily crude product. The crude product was dissolved in ethyl acetate (20 mL) and added with heptane (40 mL) to deposit crystals. Further, the crystals were recrystallized from toluene (24 mL) to obtain 5-[6-{2-fluorobenzyloxy}-2-naphthyl]-methyl-2,4-thiazolidinedione (4.30 g, yield: 62%). Physicochemical properties of the resulting 5-[6-{2-fluorobenzyloxy}-2-naphthyl]-methyl-2,4-thiazolidinedione are shown below.
   NMR (DMSO-d₆) δ:3.23 (dd, 1H, J=9.5, 14.0Hz), 3.51 (dd, 1H, J=4.3, 14.0Hz), 4.99 (dd, 1H, J=4.3, 9.5Hz), 5.24 (s, 2H), 7.20-7.30 (m, 4H), 7.38 (t, 1H, J=8.8Hz), 7.45 (s, 1H), 7.61 (t, 1H, J=7.5Hz), 7.70 (s, 1H), 7.76 (d, 1H, J=5.8Hz), 7.79 (d, 1H, J=6.0Hz), 12.03 (s, 1H)
   IR (KBr): 3254, 3055, 1759, 1674, 1607, 1493, 1393, 1325, 1269, 1231cm⁻¹
   Melting point: 150-151°C
(B) A suspension of sodium hydride (content: about 60%, 7.47 g) in DMF (500 mL) was added with the 5-(6-hydroxy-2-naphthyl)methyl-2,4-thiazolidinedione (25.00 g, 88.3 mmol) obtained above and dissolved in DMF (44 mL) and stirred at about 25°C for 2.5 hours. Then, the mixture was cooled to 15°C, added with 2-fluorobenzyl chloride (12.6 mL, 106.0 mmol) and stirred at 15°C for 6 hours. The reaction mixture was added with saturated aqueous ammonium chloride (16.5 mL) to stop the reaction and further added with heptane (320 mL). The oil was washed and separated, and the DMF layer was added to saturated aqueous ammonium chloride (2.3 L). The deposited solid was separated by filtration and dissolved in ethyl acetate (100 mL). Then, the organic layer was washed with water and concentrated under reduced pressure to obtain an oily crude product. The crude product was dissolved in ethyl acetate (100 mL) and added with heptane (200 mL) to deposit crystals. Further, the crystals were recrystallized from toluene (110 mL) to obtain 5-[6-{2-fluorobenzyloxy}-2-naphthyl]methyl-2,4-thiazolidinedione (20.0 g, yield: 59%).
(C) Under ice cooling, a solution of the 5-(6-hydroxy-2-naphthyl)methyl-2,4-thiazolidinedione (157 mg, 0.57 mmol) obtained above in DMF (6 mL) was added with sodium hydride (content: 60%, 48 mg, 12 mmol) and stirred at 10 to 15°C for 1.5 hours. Then, the mixture was added with 2,4,6-trifluorobenzyl bromide (149 mg, 0.66 mmol) and stirred at 20-25°C for 3 hours. The reaction mixture was neutralized by addition of diluted hydrochloric acid and added with ethyl acetate (30 mL) to extract the mixture. The organic layer was washed with saturated aqueous sodium hydrogencarbonate and saturated brine, dried over magnesium sulfate and concentrated under reduced pressure to obtain a crude product. The product was purified by column chromatography (ethyl acetate/heptane = 30%) (and recrystallized) to obtain 5-[6-{2,4,6-trifluorobenzyloxy}-2-naphthyl]-methyl-2,4-thiazolidinedione (97 mg, yield: 39%). Physicochemical properties of the resulting 5-[6-{2,4,6-trifluorobenzyloxy}-2-naphthyl]-methyl-2,4-thiazolidinedione are shown below.
   NMR (DMSO-d₆) δ: 3.25 (dd, 1H, J=9.0, 13.9Hz), 3.53 (dd, 1H, J=4.1, 13.9Hz), 4.99 (dd, 1H, J=4.1, 9.0Hz), 5.19 (s, 2H), 7.16-7.48 (m, 5H), 7.70-7.81 (m, 3H), 12.03 (s, 1H) IR (KBr): 3425, 3252, 1687, 1666, 1331, 1263, 1122, 927, 844, 771 cm⁻¹
   Melting point: 166-167°C
(D) 5-[6-{2,6-Difluorobenzyloxy}-2-naphthyl]-methyl-2,4-thiazolidinedione (366 mg, yield: 58%) was obtained from 5-(6 hydroxy-2-naphthyl)methyl-2,4-thiazolidinedione. (432 mg, 1.58 mmol) and 2,6-difluorobenzyl bromide (360 mg, 1.74 mmol) in the same manner as in the above (B). Physicochemical properties of this 5-[6-{2,6-difluorobenzyloxy}-2-naphthyl]-methyl-2,4-thiazolidinedione are shown below.
   NMR (DMSO-d₆) δ: 3.24 (dd, 1H, J=9.3, 14.1Hz), 3.52 (dd, 1H, J=4.2, 14.1Hz), 4,98 (dd, 1H, J=4.2, 9.0Hz), 5.22 (s, 2H), 7.17 (dd, 1H, J=3.6, 9.3Hz), 7.20 (d, 1H, J=8.1Hz), 7.38 (d, 1H, J=8.4Hz), 7.4-7.6 (m, 2H), 7.69 (s, 1H), 7.78 (m, 2H), 12.03 (brs, 1H)
   IR (KBr): 3177, 3056, 2359, 1705, 1471, 1233, 1057, 926 cm⁻¹
   Melting point: 160-161°C
(E) 5-[6-{2,6-Dichlorobenzyloxy}-2-naphthyl]-methyl-2,4-thiazolidinedione (102 mg, yield 21%) was obtained from 5-(6-hydroxy-2-naphthyl)methyl-2,4-thiazolidinedione (306 mg, 1.12 mmol) and 2,6-dichlorobenzyl bromide (540 mg, 2.24 mmol) in the same manner as in the above (B). Physicochemical properties of this 5-[6-{2,6-dichlorobenzyloxy}-2-naphthyl]-methyl-2,4-thiazolidinedione are shown below.
   NMR (DMSO-d₆) δ: 3.25 (dd, 1H, J=9.3, 14.1Hz), 3.53 (dd, 1H, J=4.2, 14.1Hz), 4.99 (dd, 1H, J=4.2, 9.3Hz), 5.34 (s, 2H), 7.18 (dd, 1H, J=2.4, 9.0Hz), 7.39 (dd, 1H, J=1.5, 8.4Hz), 7.4-7.6 (m, 4H), 7.69(s, 1H), 7.79 (d, 2H, J=9.0Hz), 12,03 (brs, 1H)
   IR (KBr): 3277, 3057, 2359, 1682, 1437, 1225, 1163, 1013 cm⁻¹
   Melting point: 165-168°C
(F) 5-[6-{2,4-Dichlorobenzyloxy}-2-naphthyl]-methyl-2,4-thiazolidinedione (195 mg, yield: 36%) was obtained from 5-(6-hydroxy-2-naphthyl)methyl-2,4-thiazolidinedione (342 mg, 1.25 mmol) and 2,4-dichlorobenzyl chloride (732 mg, 3.75 mmol) in the same manner as in the above (B). Physicochemical properties of this 5-[6-{2,4-dichlorobenzyloxy}-2-naphthyl]-methyl-2,4-thiazolidinedione are shown below.
   NMR (DMSO-d₆) δ: 3.23 (dd, 1H, J=9.6, 14.4Hz), 3.52 (dd, 1H, J=4.2, 14.4Hz), 4.98 (dd, 1H, J=4.2, 9.3Hz), 5.25 (s, 2H), 7.25 (d, 1H, J=8.9Hz), 7.3-7.4 (m, 2H), 7.50 (dd, 2H, J=1.9, 8.4Hz), 7.58 (d, 1H, J=1.7Hz), 7.6-7.7 (m, 2H), 7.80 (t, 2H, J=7.5Hz), 12.10 (brs, 1H)
   IR (KBr): 3464, 3055, 1701, 1606, 1244, 1041, 752 cm⁻¹
   Melting point: 147-149°C

### Industrial Applicability

According to the present invention, 6-hydroxy-2-naphthylcarbinol useful as synthetic materials for perfumes, resins, agricultural chemicals, drugs and the like and 6-hydroxy-2-naphthaldehyde can efficiently be produced on an industrial scale. Further, by using the compounds, 5-[6-{2-fluorobenzyloxy}-2-naphthyl]-methyl-2,4-thiazolidinedione can be industrially manufactured, which has hypoglycemic action as well as hypolipidemic action of blood lipids and is useful as medicaments for prophylactic and/or therapeutic treatment of diabetes mellitus and hyperlipidemia.

## Claims

1. 6-Hydroxy-2-naphthylcarbinol.

2. 6-Hydroxy-2-naphthylcarbinol for use as a synthetic intermediate for preparation of 6-hydroxy-2-naphthaldehyde.

3. 6-Hydroxy-2-naphthylcarbinol for use a synthetic intermediate for preparation of a compound represented by the following general formula (X): 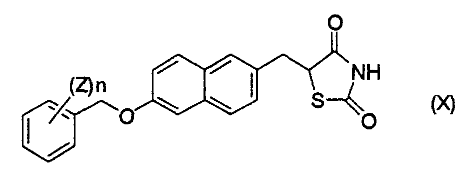 wherein Z represents a halogen atom and n represents an integer of 1 to 5.

4. A method for preparing 6-hydroxy-2-naphthylcarbinol, which comprises the step of reducing 6-hydroxy-2-naphthalenecarboxylic acid.

5. The method according to claim 4, wherein the reduction is performed by using a borane.

6. The method according to claim 5, wherein the reduction is performed in the presence of a borate represented by the following general formula (III): 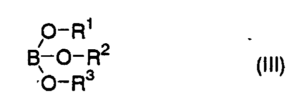 wherein R¹, R² and R³ independently represent an alkyl group, an aralkyl group, a hetero atom-containing alkyl group, or an aryl group; a borate represented by the following general formula (IV): 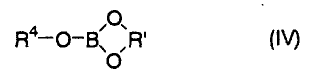 wherein R⁴ represents an alkyl group, an aralkyl group, a hetero atom-containing alkyl group, or an aryl group, and R' represents an alkylene group which may be substituted; a borate represented by the following general formula (V): 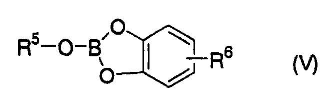 wherein R⁵ and R⁶ each independently represent an alkyl group, an aralkyl group, a hetero atom-containing alkyl group, or an aryl group; or a borate represented by the following general formula (VI): 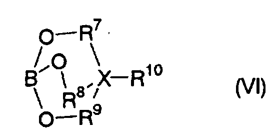 wherein R⁷, R⁸ and R⁹ independently represent an alkylene group which may be substituted or an alkyleneoxy group which may be substituted, R¹⁰ represents a hydrogen atom or an alkyl group, X represents a carbon atom, a nitrogen atom, or a boron atom, and a, b, c and d independently represent 0 or 1.

7. The method according to claim 6, wherein the borate is trimethyl borate or triethyl borate.

8. The method according to claim 4, wherein the reduction is performed by using an aluminum hydride complex compound.

9. A method for preparing 6-hydroxy-2-naphthaldehyde, which comprises the step of oxidizing 6-hydroxy-2-naphthylcarbinol.

10. A method for preparing 6-hydroxy-2-naphthaldehyde, which comprises the steps of:
(1) reducing 6-hydroxy-2-naphthalenecarboxylic acid to produce 6-hydroxy-2-naphthylcarbinol; and
(2) oxidizing the 6-hydroxy-2-naphthylcarbinol obtained in the above step (1).

11. The method according to claim 9 or claim 10, wherein the oxidation is performed in the presence of manganese dioxide.

12. The method according to claim 11, wherein the oxidation is performed in the presence of activated manganese dioxide.

13. The method according to claim 9 or 10, wherein the oxidation is performed by using oxygen in the presence of a catalyst system containing a nitroxide represented by the following general formula (VII): 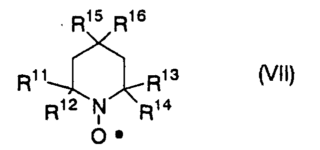 wherein R¹¹, R¹², R¹³ and R¹⁴ independently represent an alkyl group, an aryl group, or a hetero-atom substituted alkyl group, and R¹⁵ and R¹⁶ independently represent a hydrogen atom, an alkyl group, an aryl group, or a hetero atom containing substituent; or a nitroxide represented by the following general formula (VIII): 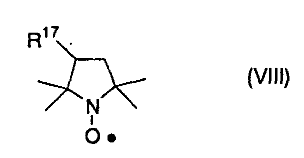 wherein R¹⁷ represents a hydrogen atom, a hydroxyl group, an alkyl group, an alkoxycarbonyl group, a cyano group, a carbamoyl group, an alkoxy group, or an acyloxy group together with a metal compound having oxidation-reduction ability or a metal complex compound having oxidation-reduction ability.

14. The method according to claim 13, wherein the nitroxide is 2,2,6,6-tetramethylpiperidine-1-oxyl or 4-hydroxy-2,2,6,6-tetramethylpiperidine-1-oxyl, and a metal of the metal compound having oxidation-reduction ability or the metal complex compound having oxidation-reduction ability is copper or iron.

15. A method for preparing a compound represented by the following general formula (X): 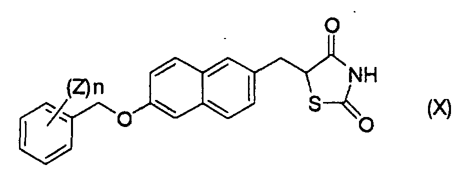 wherein Z represents a halogen atom, and n represents an integer of 1 to 5 which comprises the steps of:
(1) oxidizing 6-hydroxy-2-naphthylcarbinol to produce 6-hydroxy-2-naphthaldehyde;
(2) reacting the 6-hydroxy-2-naphthaldehyde obtained in the above step (1) with 2,4-thiazolidinedione without protection of the hydroxyl group of the 6-hydroxy-2-naphthaldehyde to produce 5-(6-hydroxy-2-naphthyl)methylene-2,4-thiazolidinedione;
(3) reducing the 5-(6-hydroxy-2-naphthyl)methylene-2,4-thiazolidinedione obtained in the above step (2) to produce 5-(6-hydroxy-2-naphthyl)methyl-2,4-thiazolidinedione; and
(4) reacting the 5-(6-hydroxy-2-naphthyl)methyl-2,4-thiazolidinedione obtained in the above step (3) with a compound represented by the following general formula (IX): 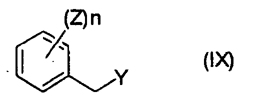
wherein Y represents a halogen atom, an alkylsulfonyloxy group, or an arylsulfonyloxy group, and Z and n have the same meanings as those defined above.

16. A method for preparing a compound represented by the following general formula (X): 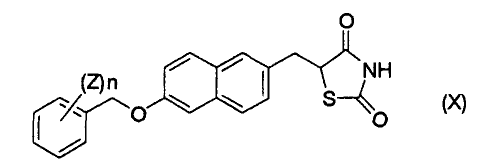 wherein Z represents a halogen atom, and n represents an integer of 1 to 5, which comprises the steps of:
(1) reducing 6-hydroxy-2-naphthalenecarboxylic acid to produce 6-hydroxy-2-naphthylcarbinol;
(2) oxidizing the 6-hydroxy-2-naphthylcarbinol obtained in the above step (1) to produce 6-hydroxy-2-naphthaldehyde;
(3) reacting the 6-hydroxy-2-naphthaldehyde obtained in the above step (2) with 2,4-thiazolidinedione without protection of the hydroxyl group of the 6-hydroxy-2-naphthaldehyde to produce 5-(6-hydroxy-2-naphthyl)methylene-2,4-thiazolidinedione;
(4) reducing the 5-(6-hydroxy-2-naphthyl)methylene-2,4-thiazolidinedione obtained in the above step (3) to produce 5-(6-hydroxy-2-naphthyl)methyl-2,4-thiazolidinedione; and
(5) reacting the 5-(6-hydroxy-2-naphthyl)methyl-2,4-thiazolidinedione obtained in the above step (4) with a compound represented by the following general formula (IX): 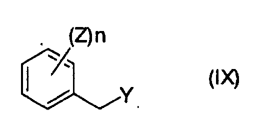
wherein Y represents a halogen atom, an alkylsulfonyloxy group, or an arylsulfonyloxy group, and Z and n have the same meanings as those defined above.

17. The method according to claim 15 or claim 16, wherein 6-hydroxy-2-naphthaldehyde and 2,4-thiazolidinedione are subjected to dehydration condensation in the presence of a base to obtain 5-(6-hydroxy-2-naphthyl)methylene-2,4-thiazolidinedione.
